# EUROPEAN PATENT SPECIFICATION

(11) **EP 2 094 830 B1**
(45) Date of publication and mention of the grant of the patent: **10.04.2013**
(21) Application number: 07866245.9
(22) Date of filing: 18.12.2007
(51) Int. Cl.: C12N 1/20, A61K 35/74, A23L 1/03

(54) **USES AND METHODS FOR PREVENTING AND/OR TREATING CARIES CAUSED BY MUTANS STREPTOCOCCI**
ANWENDUNGEN UND VERFAHREN ZUR VORBEUGUNG UND/ODER BEHANDLUNG VON DURCH MUTANS STREPTOCOCCI VERURSACHTER KARIES
MÉTHODES ET MOYENS A PRÉVENIR ET/OU TRAITER DES CARIES CAUSÉES PAR MUTANS STREPTOCOCCI

(30) Priority: 19.12.2006 EP 06026301
(43) Date of publication of application: 02.09.2009
(73) Proprietor: BASF SE, 67056 Ludwigshafen (DE)
(72) Inventor: REINDL, Andreas, 68199 Mannheim (DE); LANG, Christine, 10625 Berlin (DE); BÖTTNER, Mewes, 10407 Berlin (DE); VEEN, Markus, 84453 Altmühldorf (DE)
(86) International application number: PCT/EP2007/011127
(87) International publication number: WO 2008/074473

(56) References cited:
- EP-A- 1 312 667
- EP-A- 1 634 948
- WO-A-2004/067729
- WO-A-2006/027265
- US-A- 4 746 512
- US-A1- 2004 101 495
- SOOKKHEE S ET AL: "Lactic acid bacteria from healthy oral cavity of Thai volunteers: Inhibition of oral pathogens" JOURNAL OF APPLIED MICROBIOLOGY, vol. 90, no. 2, February 2001 (2001-02), pages 172-179, XP002321894 ISSN: 1364-5072
- KRUGER C ET AL: "In situ delivery of passive immunity by lactobacilli producing single-chain antibodies" NATURE BIOTECHNOLOGY, vol. 20, no. 7, July 2002 (2002-07), pages 702-706, XP002321895 ISSN: 1087-0156 cited in the application
- CHUNG J ET AL: "Isolation and characterization of Lactobacillus species inhibiting the formation of Streptococcus mutans biofilm." ORAL MICROBIOLOGY AND IMMUNOLOGY. JUN 2004, vol. 19, no. 3, June 2004 (2004-06), pages 214-216, XP002321896 ISSN: 0902-0055 cited in the application
- WEI H ET AL: "Stability and activity of specific antibodies against Streptococcus mutans and Streptococcus sobrinus in bovine milk fermented with Lactobacillus rhamnosus strain GG or treated at ultra-high temperature." ORAL MICROBIOLOGY AND IMMUNOLOGY, vol. 17, no. 1, February 2002 (2002-02), pages 9-15, XP002321897 ISSN: 0902-0055
- WHATMORE ADRIAN M ET AL: "Re-evaluation of the taxonomic position of Streptococcus ferus" INTERNATIONAL JOURNAL OF SYSTEMATIC AND EVOLUTIONARY MICROBIOLOGY, vol. 52, no. 5, September 2002 (2002-09), pages 1783-1787, XP002492129 ISSN: 1466-5026
- LOESCHE W J: "Role of Streptococcus mutans in human dental decay." MICROBIOLOGICAL REVIEWS, vol. 50, no. 4, December 1986 (1986-12), pages 353-380, XP002492130 ISSN: 0146-0749 cited in the application

## Description

The present invention relates to the use of a microorganism belonging to the genus Lactobacillus or a mutant or derivative (as defined below) thereof, characterized in that it is capable of specifically binding to a bacterium belonging to the group of mutans Streptococci, wherein the specific binding is (i) resistant to heat treatment, wherein said heat treatment is carried out at a temperature of more than 95 °C for at least 20 minutes; and (ii) resistant to protease treatment, wherein said protease treatment is treatment with a protease selected from the group consisting of pronase E, proteinase K, trypsin and chymotrypsin; and (iii) calcium-dependent; and (iv) formed within a pH range between 4.5 and 8.5; and (v) formed in the presence of saliva, for the preparation of an anticariogenic composition for the treatment or prevention of caries caused by Streptococcus sobrinus.

Preferably, the specific binding can be assayed as follows:
(a) growing said microorganism to stationary phase;
(b) mixing said microorganism with a bacterium belonging to the group of mutans Streptococci which has been grown to stationary phase;
(c) incubating the mixture obtained in step (b) under conditions allowing the formation of aggregates of said microorganism and a bacterium of the group of mutans Streptococci; and
(d) detecting aggregates by the occurrence of a pellet.

Another aspect in connection with the present invention is a method of prophylaxis or treatment of caries caused by mutans Streptococci other than Streptococcus mutans, comprising administering a microorganism belonging to the group of lactic acid bacteria characterized in that said microorganism is capable of specifically binding to a bacterium belonging to the group of mutans Streptococci or a mutant, derivative or fragment of said microorganism.

Mutans Streptococci colonize the host after the first teeth erupt (Carlson et al., Caries Res. 9 (1975), 333-339). They are localized on the surfaces of the teeth, and their abundance in the plaque is highest over intitial leisions (Duchin and van Houte Arch. Biol. Biol. 23 (1978) 779-786). Their level of colonization within the plaque is increased by sucrose consumption (Staat et al., J. Dent. Res. 54 (1975) 872-880). They are able to synthesize certain macro-molecules from sucrose that foster their attachment to the teeth (Tanzer et al., Infect. Immun. 10 (1974) 197-203). Mutans Streptococci are rapid producers of acid from simple carbohydrates, including sucrose, and are tolerant to low pH (Edwardsson, Arch. Biol. 13 (1968) 637-646). Furthermore, they are essentially alwas recovered on cultivation of intial and established carious lesion sites (Littleton et al., Arch. Oral. Biol. 15 (1979) 461-463). Interest in them grew after the demonstration of their potent induction and progression of carious lesions in a variety of experimental animals, including mono-infected gnotobiotes. Their virulence expression is strongly associated with consumption of carbohydrates, especially sucrose.

The role of further bacterial species that are connected to caries development like lactic acid bacteria or actinomycetes is not conclusive. These bacteria are often found in cariotic lesions, but only in association mutans Streptococci. According to present knowledge the presence of mutans Streptococci is an indispensable condition of cariogenesis (Tanzer et al., J. Dent. Educ. 65 (2001) 1028-1037). The group of mutans Streptococci has been defined as comprising at least S. mutans, S. sobrinus, S. cricetus, S. ratti, S. ferus and S. macacae (Loesche et al., Microbio. Rev. 50 (4) (1986) 353-380). Due to the fact that Streptococcus mutans is the most abundant representative of mutans Streptococci in humans, most microbiological caries research as well as anti-caries measures concentrate on this specific species.

The initial binding of S. mutants to the surface of the teeth occurs via two mechanisms. The first mechanism is binding of S. mutans via the streptococcal antigen I/II (SA I/II) - a surface protein also known by the synonyms B, IF, P1, SR, MSL-1 or PAc - to the pellicle, a layer of saliva proteins on the teeth surface. Antibodies against this protein have been shown to prevent the adhesion of S. mutans in vitro.

Accordingly, the streptococcal antigen I/II (SA I/II) is a target for vaccination. In different recombinant combinations - the complete antigen, the saliva binding region, the protein coupled to cholera toxin or expressed on the surface of an avirulent Salmonella strain - a successful immunization of animals has been shown. This resulted in high IgA titers and a reduction of S. mutans colonization (Huang et al., Infect. Immun. 69 (2001), 2154-2161). Comparable results have been achieved using a DNA-vaccine coding for SA I/II (Fan et al., J. Dent. Res. 81 (2002), 784 - 787). Passive immunity has been achieved by recombinant expression of anti-SA I/II antibodies on the surface of lactic acid bacteria. These lactobacilli aggregate S. mutans and administration of the bacteria to rats led to a reduction of caries development (Krueger et al., Nature Biotechnology 20 (2002), 702 - 706).

WO 06/027265 provides lactic acid bacteria capable of binding to S. mutans with the aim to suppress adhesion to the teeth.

The most important binding partner of the streptococcal antigen is the salivary agglutinin, a protein similar to the lung glycoprotein gp-340 from the scavenger receptor cysteine-rich superfamily (Prakobphol et al., J. Biol. Chem. 275 (2000) 39860-39866).

The role of agglutinin in cariogenesis is not entirely understood so far. It can lead to the adhesion of S. mutans when present bound to surfaces, and it can lead to an aggregation of S. mutans when present in a soluble state. The latter might result in a removal of aggregated S. mutans from the mouth by saliva flow. A high agglutinin concentration in saliva leads in vitro to an increase in the adhesion of S. mutans, whereas in vivo there is no clear correlation between the agglutinin concentration in saliva and the risk for caries (Stenudd et al., J. Dent. Res. 80 (2001), 2005-2010).

Monoclonal antibodies against agglutinin completely block the binding of S. mutans to saliva-coated hydroxyapatite in vitro and prevent the agglutinin dependent aggregation (Carlen und Olsson, J. Dent. Res. 74 (1995), 1040-1047; Carlen et al., J. Dent. Res. 77 (1998), 81-90). Brady et al., Infect. Immun. 60 (1992), 1008-1017 showed that the surface adhesion and the aggregation can be independently inhibited by different antibodies. This indicates that different epitopes of agglutinin are responsible for these two effects.

Other saliva proteins frequently connected to the development of caries are proline-rich proteins (PRPs). However, the role of these proteins in the adhesion of cariogenic bacteria is discussed controversially. These proteins are coded by two gene loci (PRH-1 and PRH-2) and occur in different variants that differ in only a few amino acids (PRP-1, PRP-2, PIF. Db-double band). These variants can be cleaved proteolytically, resulting in the so-called small PRPs (PRP-3, PRP-4. PIF-f and Db-f). PRPs mediate a strong binding of commensales like Actinomyces naeslundii or non-mutans streptococci. Interestingly, this binding takes place only after adhesion of the protein to the tooth surface, resulting in a conformational shift making the binding sites accessible. S. mutans is only weakly bound. The PRP-variant Db is of relevance for the effective binding of S. mutans. A high concentration of Db correlates with a high adhesion of S. mutants and a strong development of caries. A reduced part of PRP-Db of a high total PRP concentration correlates with a low development of caries (Stenudd et al., J. Dent. Res. 80 (2001), 2005-2010). It is unknown, if S. mutans binds directly to PRPs.

The second way of S. mutans to adhere to the tooth surface is via a sucrose dependent adhesion. S. mutans expresses three different glycosyltransferases (GTFs) that are capable of synthesizing the sugar polymer glucan. Glucans exist in a water soluble form (1-6 glycosidic linkage) and a non-soluble form called mutan (1-3 glycosidic linkage). Mutan cannot be degraded either by oral bacteria or by enzymes in saliva. It forms a sticky matrix within the dental plaque that is the basis for the sucrose dependent adhesion of S. mutans. The glycosyltransferases GTFB and GTFC, the prevalent enzymes responsible for mutan formation, are located on the cell surface of S. mutans. In contrast, the glycosyltransferase GTFD synthesises the soluble glucan and is secreted by S. mutans. Experiments using GTF deficient mutants of S. mutans show that an interaction of all three enzymes is necessary for a sucrose dependent adhesion (Ooshima et al., J. Dent. Res. 80 (2001), 1672-1677). Glycosyltransferases have an N-terminal sucrose binding site and a C-terminal glucan binding site. Antibodies against the enzyme or against the glucan binding site lead to an inhibition of the sucrose dependent adhesion of S. mutans. It has not been possible to block the N-terminal sucrose binding site using antibodies (Yu et al., Infect. Immun. 65 (1997), 2292-2298).

An inhibition of glycosyltransferases followed by a reduced adhesion of S. mutans can also be achieved by some flavonoids or terpenoids (US 2004/0057908) or propolis extracts (Duarte et al., Biol. Pharmacol. Bull. 26 (2003), 527 - 531).

Lactic acid bacteria named S11 have been found, that reduce mutan formation and, therefore, adherence of S. mutans in vitro. As described above, mutan formation is essential for S. mutans to adhere to the tooth surface. Accordingly, Chung et al. (Oral Microbiol. Immunol. 19 (2004), 214 - 216) have found detached S. mutans cells when they have been incubated with the lactic acid bacteria of strain S11 which are said to reduce mutan formation. The binding of S. mutans to mutan occurs via bacterial binding proteins (glucan binding protein). The exact mechanism of this binding has to be determined (Sato et al., Infect. Immun. 65 (1997), 668-675).

The fungi Trichoderma harzianum and Penicillium purpurogenum produce homologous alpha-1,3-glucanases (Fuglsang et al., J. Biol. Chem. 275 (2000), 2009-2018). The use of Enterococcus, Lactobacillus and Lactococcus species effective against glucan production and plaque formation is described (US 6.036.952). The mechanism of action has to be elucidated.

A further approach to inhibit caries is to neutralise the low pH in the plaque. Urea and arginine are components of saliva. Urea is present in concentrations of 3 - 10 mmol/L without major differences between caries free and caries affected persons. The concentration of free arginine differs between 4 and 40 µmol/L. Caries free individuals have a higher average of free arginine concentrations in saliva than caries affected persons (van Wuyckhuyse et al., J. Dent Res. 74 (1995), 686-690).

Some plaque bacteria like Streptococcus sanguis and Actinomyces naeslundi are capable of cleaving urea or arginine resulting in the formation of ammonia. The alkaline ammonium rises the pH of the plaque and therefore reduces caries (Curran et al., Appl. Environm. Microbiol. 61 (1995), 4494-4496; Morou-Bermudez and Bume, Infect. Immun. 68 (2000), 6670-6676). Accordingly, these bacteria are suggested to be used to treat caries. Another approach suggested for treating caries is that by proteolyses of PRP-1 and PRP-3 arginine rich peptides are created, that can, after further proteolysis by oral bacteria like S. sanguis, S. oralis and S. mitis, lead to a higher pH in the plaque. By application of a recombinant variant of these peptides, the sucrose dependent decrease of the pH is inhibited (Li et al., Infect. Immun. 68 (2000), 5425-5429). Moreover, it is described that by using a urea containing chewing gum after sucrose intake the drop of pH can be inhibited and, accordingly, for example, S. mutans may not contribute so much to caries.

However, as is evident from the above, the prior art concentrates on anti-caries measures directed against Streptococcus mutans. No measures are described that would allow to target mutans Streptococci other than Streptococcus mutans despite their possible role in cariogenesis. Hence, there is a need for means and methods which fulfil the aforementioned desirable criteria and which are useful for preventing and/or treating caries caused by bacteria other than Streptococcus mutans.

The technical problem underlying the present invention is thus to comply with the above described needs. The solution to said technical problem is achieved by providing the embodiments as characterized in the claims.

Accordingly, in a first aspect the present invention relates to the use of a microorganism belonging to the genus Lactobacillus or a mutant or derivative thereof, characterized in that it is capable of specifically binding to a bacterium belonging to the group of mutans Streptococci, wherein the specific binding is (i) resistant to heat treatment, wherein said heat treatment is carried out at a temperature of more than 95 °C for at least 20 minutes; and (ii) resistant to protease treatment, wherein said protease treatment is treatment with a protease selected from the group consisting of pronase E, proteinase K, trypsin and chymotrypsin; and (iii) calcium-dependent; and (iv) formed within a pH range between 4.5 and 8.5; and (v) formed in the presence of saliva, for the preparation of an anticariogenic composition for the treatment or prevention of caries caused by Streptococcus sobrinus,
wherein said derivative of the microorganism is an inactivated form of said microorganism or a fragment of said microorganism, said microorganism being thermally inactivated or lyophilized, wherein said fragment is a membrane fraction obtained by a membrane preparation and wherein said inactivated form or said fragment retains the capability of specifically binding a bacterium belonging to the group of mutans Streptococci.

Preferably, the specific binding can be assayed as follows:
(a) growing said microorganism to stationary phase;
(b) mixing said microorganism with a bacterium belonging to the group of mutans Streptococci which has been grown to stationary phase;
(c) incubating the mixture obtained in step (b) under conditions allowing the formation of aggregates of said microorganism and a bacterium of the group of mutans Streptococci; and
(d) detecting aggregates by the occurrence of a pellet.

The bacterium belonging to the group of mutans Streptococci used in such an assay can be Streptococcus mutans.

The specific binding is preferably assayed as described in Example 4 herein below.

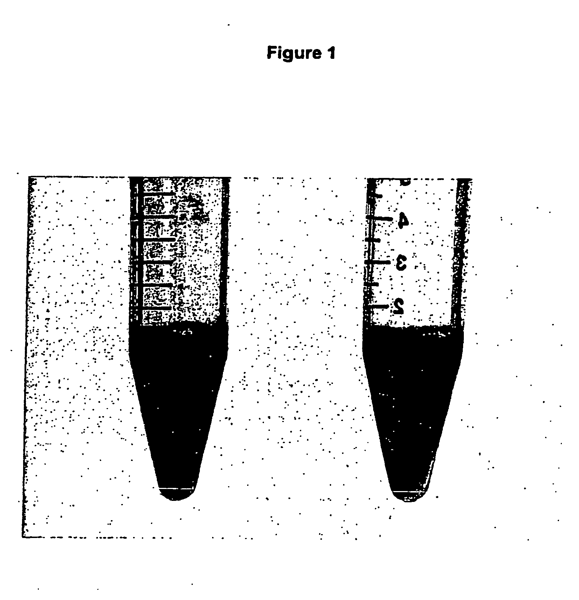

In particular, for a pelleting aggregation assay of mutans Streptococci as described in Example 4, infra, microorganisms belonging to the group of lactic acid bacteria, namely microorganisms belonging to the genus Lactobacillus, are preferably mixed with mutans Streptococci in volumetric ratios of 3:1 to 60:1 (mutans Streptococci: lactobacilli). Both, the lactic acid bacteria and mutans Streptococci are grown to stationary phase as described in Example 1. Preferably, the optical density is measured photometrically at a wavelength of 600 nm. The mentioned ratios correspond to a ratio of colony forming units from 1:50 to 1:2.5. Preferably, an OD₆₀₀=1 in 1 ml correlates to 3 x 10⁸ colony forming units of a mutans Streptococcus. Preferably, an OD₆₀₀=1 in 1 mL correlates to 7 x 10⁹ colony forming units of lactobacilli as described herein below. Preferably, for assaying the aggregation reaction by pelleting, the bacteria are in a volume of 2 ml in 15 ml Falcon tubes. If necessary, the culture suspensions are diluted with PBS-butter to obtain volumetric ratios mentioned above, while keeping the final volume at 2 ml. Preferably, the mixture is vortexed for about 15 seconds and then left undisturbed for at least 5, 10, 15 minutes and more preferably for at least 20 minutes at room temperature, i.e. any temperature between 16°C and 25°C. An aggregation is visible as an immediate turbity of the suspension and, after at least 20 minutes an aggregation is visible by aggregates that settle as a visible pellet (exemplarily shown in Figure 1, left Falcon tube), whereas non-mutans Streptococcus aggregating mixtures stay in suspension (exemplarily shown in Figure 1, right Falcon tube). As a control, self-aggregation of the respective lactic acid bacterium and the mutans Streptococcus strain can be assayed by omitting either the mutans Streptococcus or the lactic acid bacterium.

The aggregation of a lactobacillus and a mutans Streptococcus according to the above described assay can be quantified by separating the formed aggregates by centrifugation, e.g. at 500 x g for 30 seconds. Subsequently, the amount of aggregation can be determined by measuring the amount of non-aggregated cells that are left in the supernatant The determination can be carried out by any suitable means known to the person skilled in the art. Preferably, the determination is carried out by removing a certain volume of the supernatant, e.g. 1 ml. Subsequently, the optical density of the removed supernatant may be measured at any suitable wavelength, known to the skilled person, e.g. at 600 nm. The measured value after subtraction of a value a corresponding control test without lactobacilli represents the amount of cells that have not been aggregated.

Alternatively, in order to address the possible problem of self-aggregation a stain, preferably a fluorescent stain, can be employed. The specific binding can be assayed as follows:
(a) growing said microorganism to stationary phase;
(b) mixing said microorganism with a bacterium belonging to the group of mutans Streptococci which has been grown to stationary phase and which has been stained using a suitable stain, preferably a fluorescent stain;
(c) incubating the mixture obtained in step (b) under conditions allowing the formation of aggregates of said microorganism and a bacterium of the group of mutans Streptococci; and
(d) detecting aggregates by the detection of the stain, preferably a fluorescencent stain.

The bacterium belonging to the group of mutans Streptococci used in such an assay can be Streptococcus mutans. Preferably such an aggregation assay may be carried out as described in Example 5, herein below. In particular, for an aggregation assay of mutans Streptococci as described in Example 5, infra, both, the lactic acid bacteria, namely microorganisms belonging to the genus Lactobacillus, and mutans Streptococci are grown to stationary phase as described in Example 1. Preferably, the optical density is measured photometrically at a wavelength of 600 nm. Preferably, an OD₆₀₀=1 in 1 ml correlates to 3 x 10⁸ colony forming units of a mutans Streptococcus. Preferably, an OD₆₀₀=1 in 1 mL correlates to 7 x 10⁹ colony forming units of lactobacilli as described herein below. Subsequently, the mutans Streptococci are stained. In a further preferred embodiment, the lactobacilli are stained, whereas the Streptococci are not stained. As stain any suitable stain can be used, preferably a fluorescence stain known to the person skilled in the art may be used. Preferably, a specific or unspecific fluorescence stain may be used, for example, CFDA-SE. Specfically, the cells are harvested, e.g. by centrifugation, preferably at 3200 x g for 5 min. Subsequently, the obtained pellet may be resuspended in any suitable buffer known the person skilled in the art, preferably in a PBS-buffer. The amount of buffer may be calculated so that the resulting suspension has an OD600 of, e.g., 4.2/ml. Subsequently, the suspension may be mixed with a suitable stain, e.g. a fluorescence stain, preferably with 5,6-carboxyfluorescein diacetate, succhinimidyl ester (CFDA-SE), more preferably with 2µl of a CFDA-SE solution (Invitrogen). Subsequently, the cells may be incubated for a suitable time period, as known to the skilled person, e.g. for 2 hours, at a suitable temperature as known to the skilled person, for instance, at 37°C. In a further step, the stained cells may be harvested, e.g. by centrifugation. Preferably, the centrifugation is carried out at 3200 x g for 5 min. The cells may then be resuspended in a suitable buffer, as known to the person skilled in the art, e.g. in 2 ml of a PBS-buffer. For the aggregation, microorganisms belonging to the group of lactic acid bacteria are preferably mixed with mutans Streptococci in volumetric ratios of 3:1 to 1:3 (mutans Streptococci: lactobacilli). More preferably, the volumetric ratio of the mixture is 1:1. The mentioned ratios correspond to a ratio of colony forming units from 1:50 to 1:150. For assaying the aggregation reaction via measuring the staining, preferably the fluorescence, the lactobacilli and the mutans Streptococci are used in any suitable volume known to the skilled person, preferably, in a volume of 50µl. Preferably, the mixture is carried out in a microtiterplate, e.g. in a 96 well microtiter plate. Subsequently, the mixture may be vortexed, preferably for 12 min at full speed. Afterwards, the mixture may be centrifuged, e.g. for 10 seconds at 500 x g. The supernatant may then be removed and the pellet may be resuspended in any suitable buffer known the person skilled in the art, preferably in PBS-buffer in any suitable volume, e.g. in 100µl. The staining of the suspension may be measured in the mixture by any suitable means known to the skilled person. Preferably, in case of fluorescence, the fluorescence may be detected in a fluorescence reader, e.g. at a wavelength of 495 nm for excitation and 525 nm for emission. As controls, lactobacilli alone and stained mutans Streptococci alone may be assayed. Any background staining, e.g. fluorescence, may be measured for the tested mutans Streptococci alone and may preferably be subtracted from the value for the aggregation with the respective lactobacillus. An aggregation effect is present if the background staining, e.g. fluorescence, measured as indicated herein above, is subtracted from the measured staining, e.g. fluorescence, in a sample containing a lactobacillus as described herein above and a tested mutans Streptococcus, as described herein above, and the resulting value is at least above zero. More preferably, an aggregation effect is present if the resulting value is reproducibly above zero in a series of tests, carried out as described herein above. A "series of experiments" means at least 2, preferably 3, more preferably 4 and most preferably 5 tests.

The above described specific binding does not require magnesium. This characteristic can be tested as described in the appended Examples.

As shown in the appendend Examples, it has surprisingly been found that specific lactic acid bacteria which had originally been identified and isolated by the above mentioned binding assays because of their capacity to bind Streptococcus mutans white not binding the other Streptococcus species S. salivarius, S. oralis, S.mitis and/or S. sanguinis (see WO 06127265), show the capacity to aggregate various other mutans Streptococci, e.g. Streptococcus sobrinus, Streptococcus cricetus, Streptococcus ratti, Streptococcus ferus and Streptococcus macacae. Thus, the identification of lactic acid bacteria which show the above described binding characteristics in relation to Streptococcus mutans and wherein the binding is preferably assessed by the above-described assays provides lactic acid bacteria which do not only have the capability to aggregate Streptococcus mutans but which also have the capacity to also aggregate other mutans Streptococci, i.e. Streptococcus species which have the common characteristics as described further below and which are also brought into connection with caries development. Thus, lactic acid bacteria, which show the above recited binding characteristics versus one type of bacterium belonging to the group of mutans Streptococci (e.g. Streptococcus mutans) have been found to be able to aggregate also other bacteria belonging to the group of mutans Streptococci and can, thus, be used to prevent and/or treat caries caused by such other bacteria. Thus, the above-identified assays for testing the binding of lactic acid bacteria to one bacterium belonging to the group of mutans Streptococci allow to identify lactic acid bacteria which also bind to/aggregate other bacteria of the group of mutans Streptococci. Thus, the present invention provides an important improvement in providing means and methods for addressing caries, which is caused by mutans Streptococci other than Streptococcus mutans, namely S. sobrinus.

As is evident from the above, all the above-mentioned characteristics render the above mentioned microorganism belonging to the group of lactic acid bacteria a suitable agent for preventing and/or treating caries, in particular caries which is caused by S. sobrinus. Accordingly, the above mentioned microorganism belonging to the group of lactic acid bacteria of the genus Lactobacillus, exerts an anticariogenic effect and is thus a useful agent for preventing and/or treating caries, in particular caries caused by S. sobrinus. "Caries" or "dental caries" or "cavity" are interchangeable terms for a chronic infectious disease associated with soft decayed area in a tooth which progressively leads to the death of a tooth. It usually occurs in children and young adults but can affect any person. It is the most important cause of tooth loss in younger people. Caries can be diagnosed by methods known in the art (see, for example, Angmar-Mansson and ten Bosch, Adv. Dent. Res. 7 (1993), 70-79).

The term "mutans Streptococcus" refers to a microorganism of the taxonomic group of Streptococcus, which is found in plaque in the oral cavity and which ferments mannitol and sorbitol. Preferably, it is a microorganism with thte above mentioned characteristics which furthermore is capable to produce extracellular glucans from sucrose. Preferably, said microorganism is cariogenic, in particular in human and/or in animal models. More preferably, the term relates to a microorganism, which shows all the above-mentioned characteristics. The fermentation of sorbitol and mannitol can be tested by using any suitable test known to the person skilled in the art, for instance an API 20 Strep test (Biomerieux, France).

More preferably, the term relates to a microorganism belonging to the species Streptococcus mutans, Streptococcus sobrinus, Streptococcus cricetus, Streptococcus ratti, Streptococcus ferus or Streptococcus macacae. Even more preferably, the term relates to a microorganism belonging to Streptococcus mutans serotype c (DSMZ 20523) Streptococcus mutans serotype e (NCTC 10923) Streptococcus mutans serotype f (NCTC 11060), Streptococcus sobrinus DSM 20742, Streptococcus ratti DSM 20564, Streptococcus cricetus DSM 20562, Streptococcus ferus DSM 20646 or Streptococcus macacae DSM 20714. The term "mutans Streptococci" refers to at least one microorganism of the group of mutans Streptococcus, as described herein above. Preferably, the term refers to any combination and subgrouping of microorganisms belonging to the group of mutans Streptococcus as described herein above.

The term "preventing caries" includes prophylaxis of caries. Accordingly, a subject who has never been encountered with mutans Streptococci, the causative agents of caries, but is at a risk of being encountered, i.e. infected with mutans Streptococci benefits, for example, from the uses and methods of the present invention insofar as said subject will not suffer from caries. Hence, the uses and methods of the present invention may, for example, be applied to infants, children or young animals for prophylaxis of caries since the infant's or young animal's oral cavity is normally free of mutans Streptococci. However, the compositions as used in accordance with the present invention are not limited to administration to infants, children or young animals.

The term "treating caries" includes administration of the compositions as described herein below to a subject suffering from caries for the purpose of diminishing the amount of cells of mutans Streptococci and/or for completely depleting mutans Streptococci from the mouth, in particular from the oral cavity including the teeth. Of course, after having been cured from mutans Streptococci, it is envisaged that the respective subject benefits from the uses and methods of the present invention as regards a prophylactic anti-caries effect exerted on mutans Streptococci.

Optionally, the above mentioned microorganism belonging to the group of lactic acid bacteria is a probiotic microorganism which has, besides its anticariogenic effects, beneficial effects to the host organism to which it is administered. A "probiotic", by the generally accepted definition, is a "live microbial feed supplement which beneficially affects the host animal by improving its intestinal microbial balance".

Mutans Streptococci occur as part of the normal flora in the mouth. They are involved in the cause of dental caries in humans and animals, in particular mammals. Dental plaque adheres to the fissures and pits of the teeth adjacent to the gums. It consists initially of glycoprotein, which is precipitated and is adsorbed onto the tooth enamel. Oral bacteria then become associated with the glycoprotein. Dietary sucrose is an important contributor to caries production, particularly if the sucrose is in the form of sticky sweet foods some of which can remain in the mouth for some time. The sucrose is thus more completely metabolised by mutans Streptococci to form acid. Drinks, which contain sucrose are swallowed and so the sucrose spends less time in the mouth. It is essential that dental plaque is controlled by the use of regular tooth-brushing and the use of toothpicks and dental floss. The addition of 1 ppm of fluoride to drinking water has proved very effective in reducing caries. Moreover, the possibility of using a vaccine against Streptococcus mutans was contemplated in the scientific community. Thus, besides general schemes of oral hygiene, which affect most of the bacteria in the oral cavity, the prior art almost entirely focuses on specific measures against Streptococcus mutans. However, by the surprising finding of the present invention that naturally-occurring microorganisms belonging to the group of lactic acid bacteria, specifically to the genus of Lactobacillus, which have been identified by their capability to bind Streptococcus mutans, also have the capability to bind mutans Streptococci strains like e.g. Streptococcus sobrinus, Streptococcus cricetus, Streptococcus ratti, Streptococcus ferus or Streptococcus macacae, it is now possible to effectively prevent and/or treat caries caused by mutans Streptococci other than Streptococcus mutans, in particular S. sobrinus, since the above mentioned microorganisms belonging to the group of lactic acid bacteria are capable of aggregating and flushing away mutans Streptococci like, e.g. Streptococcus sobrinus, Streptococcus cricetus, Streptococcus ratti, Streptococcus ferus or Streptococcus macacae. Accordingly, the present invention provides the use of easily administrable bacteria, which are food-grade organisms that may, in addition to their anticariogenic properties, be useful as probiotics.

In particular, when analyzing microorganisms belonging to the group of lactic acid bacteria, specifically to the genus of Lactobacillus, which had originally been identified because of their capacity to bind Streptococcus mutans but not Streptococcus salivarius, Streptococcus oralis, Streptococcus mitis and Streptococcus sanguinis it has surprisingly been found that said microorganisms are not only capable of binding to Streptococcus mutans, but can also bind other mutans Streptococci species, like e.g. Streptococcus sobrinus, Streptococcus cricetus, Streptococcus ratti, Streptococcus ferus or Streptococcus macacae, which are the causative agents of caries. By binding to these mutans Streptococci, the microorganism belonging to the group of lactic acid bacteria, specifically to the genus of Lactobacillus as described herein, inter alia, bind to and aggregate a mutans Streptococcus and thus, in consequence, flushes away a mutans Streptococcus by the natural flow of salivary, thereby preventing and/or treating caries. On top of this, the above mentioned microorganisms belonging to the group of lactic acid bacteria do preferably not bind other microorganisms which do not belong to the group of mutans Streptococci, present in the oral cavity as is described herein and in particular in Example 6 herein below. Thus, the microenvironment of the oral cavity is not disturbed since only mutans Streptococci as the causative agents of caries are depleted. To the best knowledge, mutans Streptococci do not have any beneficial effects on the oral cavity and, thus, their loss has no adverse effect to the respective host.

Strikingly, the specific binding of the above mentioned microorganism belonging to the group of lactic acid bacteria, specifically of the Lactobacillus species described herein, to mutans Streptococci is resistant to heat treatment and/or resistant to protease treatment. In addition, the specific binding is dependent on calcium and/or independent of magnesium and stable at an acidic point of 4.5 and it occurs in the presence of saliva which renders it in particular suitable for the use in the form of oral applications or as an additive for food, feed or drinks which may contain higher concentrations of calcium, such as milk. Remarkably, thermally inactivated or lyophilised derivatives or (a) fragment(s) of said microorganisms disclosed herein are still capable of specifically binding to mutans Streptococci. This surprising effect is advantageous for using said fragment(s) of said microorganisms as well as mutants or derivatives thereof in compositions for use in animals, preferably, humans or mammals, to prevent and/or treat caries. In particular said derivatives or fragments can be easily added to any composition, e.g. cosmetic or pharmaceutical composition, food or feedstuff or drinks and the like. Additionally, the production of such derivatives or fragments is cheap and easy and they can be stored for prolonged periods of time without loosing their capability to specifically bind to mutans Streptococci. A further advantage of the above mentioned microorganism belonging to the group of lactic add bacteria is that it retains its capability to specifically bind to mutans Streptococci if it is lyophilised or spray-dried or dried. This makes it a favourable ingredient for use in the compositions described herein.

Other advantages of the invention are set forth in part in the description herein, and in part, may be obvious from the description, or may be learned from the practice of the invention.

Before the present invention is described in detail, it is to be understood that this invention is not limited to the particular methodology, protocols, bacteria, vectors, and reagents etc. described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel. B. and Kölbl, H. eds. (1995), Helvetica Chimica Acta, CH-4010 Basel, Switzerland). Throughout this specification and the claims which follow, unless the context requires otherwise, the word "comprise", and variations such as °comprises° and "comprising", will be understood to imply the inclusion of a stated integer or step or group of integers or steps but not the exclusion of any other integer or step or group of integer or step.

It must be noted that as used herein and in the appended claims, the singular forms "a", "an", and "the", include plural referents unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.

When used in the context of the present invention, the term "microorganism belonging to the group of lactic acid bacteria "encompasses (a) microorganism(s) which belong(s) to bacteria, in particular belonging to gram-positive fermentative eubacteria, more particularly belonging to the family of lactobacteriaceae including lactic acid bacteria. In addition, said term also encompasses derivatives or mutants or fragments, such as a membrane fraction as described herein, of said microorganims(s), which retain the capability to specifically bind to mutans Streptococci. The terms "derivative", "mutants" and "fragments" are described elsewhere herein. Lactic acid bacteria are from a taxonomical point of view divided up into the subdivisions of Streptococcus, Leuconostoc, Pediococcus and Lactobacillus. The above mentioned microorganism belonging to the group of lactic acid bacteria is preferably a Lactobacillus species. Members of the lactic acid bacteria group normally lack porphyrins and cytochromes, do not carry out electron-transport phosphorylation and hence obtain energy only by substrate-level phosphorylation. I.e. in lactic acid bacteria ATP is synthesized through fermentation of carbohydrates. All of the lactic acid bacteria grow anaerobically, however, unlike many anaerobes, most lactic acid bacteria are not sensitive to oxygen and can thus grow in its presence as well as in its absence. Accordingly, the above mentioned microorganisms belonging to the group of lactic acid bacteria are preferably aerotolerant anaerobic lactic acid bacteria, preferably belonging to the genus of Lactobacillus. As described above, the microorganisms used according to the present invention as described in the claims belong to the genus Lactobacillus.

The above mentioned lactic acid bacteria are preferably rod-shaped or spherical, varying from long and slender to short bent rods, are moreover preferably immotile and/or asporogenous and produce lactic acid as a major or sole product of fermentative metabolism. The genus Lactobacillus to which the above mentioned microorganism belongs is divided up by the following characteristics into three major subgroups, whereby it is envisaged that the above mentioned Lactobacillus species can belong to each of the three major subgroups:
(a) homofermentative lactobacilli
   (i) producing lactic acid, preferably the L-, D- or DL-isomer(s) of lactic acid in an amount of at least 85% from glucose via the Embden-Meyerhof pathway;
   (ii) growing at a temperature of 45°C, but not at a temperature of 15°C;
   (iii) being long-rod shaped; and
   (iv) having glycerol teichoic acid in the cell wall;
(b) homofermantative lactobacilli
   (i) producing lactic acid, preferably the L- or DL-isomer(s) of lactic acid via the Embden-Meyerhof pathway;
   (ii) growing at a temperature of 15°C, showing variable growth at a temperature of 45°C;
   (iii) being short-rod shaped or coryneform; and
   (iv) having ribitol and/or glycerol teichoic acid in their cell wall;
(c) heterofermentative lactobacilli
   (i) producing lactic acid, preferably the DL-isomer of lactic acid in an amount of at least 50% from glucose via the pentose-phosphate pathway;
   (ii) producing carbondioxide and ethanol
   (iii) showing variable growth at a temperature of 15°C .or 45°C;
   (iv) being long or short rod shaped; and
   (v) having glycerol teichoic acid in their cell wall.

Based on the above-described characteristics, the above mentioned microorganisms can be classified to belong to the group of lactic acid bacteria, particularly to the genus of Lactobacillus. By using classical systematics, for example, by reference to the pertinent descriptions in "Bergey's Manual of Systematic Bacteriology" (Williams & Wilkins Co., 1984), a microorganim can be determined to belong to the genus of Lactobacillus. Alternatively, the microorganisms can be classified to belong to the genus of Lactobacillus by methods known in the art, for example, by their metabolic fingerprint, i.e. a comparable overview of the capability of such (a) microorganism(s) to metabolize sugars or by other methods described, for example, in Schleifer et al., System. Appl. Microb., 18 (1995). 461-467 or Ludwig et al., System. Appl. Microb., 15 (1992), 487-501. The above mentioned microorganisms are capable of metabolizing sugar sources, which are typical and known in the art for microorganisms belonging to the genus of Lactobacillus. Preferably, however, the above mentioned microorganism has a metabolic fingerprint selected from the group consisting of:
(i) it metabolizes D-lactose, but not L-sorbose and/or D-saccharose and/or D-inuline,
(ii) it metabolizes inuline,
(iii) it metabolizes L-sorbose, but not D-lactose and/or D-saccharose and/or inuline, and
(iv) it metabolizes L-sorbose, D-lactose and inuline.

Preferably, the above mentioned microorganism has a metabolic fingerprint selected from the group consisting of:
(i) it metabolizes D-lactose, but not L-sorbose, D-saccharose and inuline,
(ii) it metabolizes L-sorbose, D-lactose and inuline, but not D-saccharose,
(iii) it metabolizes L-sorbose, but not D-lactose, D-saccharose and inuline, and
(iv) it metabolizes L-sorbose, D-lactose, D-saccharose, but not inuline.

Of course, the above mentioned microorganism is not limited to the metabolization of the sugars mentioned in the aforementioned metabolic fingerprint pattern, but may be capable of metabolizing further sugars which are commonly metabolized by Lactobacillus species.

The affiliation of the above mentioned microorganisms to the genus of Lactobacillus can also be characterized by using other methods known in the art, for example. using SDS-PAGE gel electrophoresis of total protein of the species to be determined and comparing them to known and already characterized strains of the genus Lactobacillus. The techniques for preparing a total protein profile as described above, as well as the numerical analysis of such profiles, are well known to a person skilled in the art. However, the results are only reliable insofar as each stage of the process is sufficiently standardized. Faced with the requirement of accuracy when determining the attachment of a microorganism to the genus of Lactobacillus, standardized procedures are regularly made available to the public by their authors such as that of Pot et al., as presented during a "workshop" organized by the European Union, at the University of Ghent, in Belgium, on Sep. 12 to 16, 1994 (Fingerprinting techniques for classification and identification of bacteria, SDS-PAGE of whole cell protein). The software used in the technique for analyzing the SDS-PAGE electrophoresis gel is of crucial importance since the degree of correlation between the species depends on the parameters and algorithms used by this software. Without going into the theoretical details, quantitative comparison of bands measured by a densitometer and normalized by a computer is preferably made with the Pearson correlation coefficient. The similarity matrix thus obtained may be organized with the aid of the UPGMA (unweighted pair group method using average linkage) algorithm that not only makes it possible to group together the most similar profiles, but also to construct dendograms (see Kersters, Numerical methods in the classification and identification of bacteria by electrophoresis, in Computer-assisted Bacterial Systematics, 337-368, M. Goodfellow, A. G. O'Donnell Ed., John Wiley and Sons Ltd, 1985).

Alternatively, the affiliation of said microorganisms to the genus of Lactobacillus can be characterized with regard to ribosomal RNA in a so- called Riboprinter.RTM. More preferably, the affiliation of the above mentioned species to the genus Lactobacillus is demonstrated by comparing the nucleotide sequence of the 16S ribosomal RNA of said bacteria, or of their genomic DNA which codes for the 16S ribosomal RNA, with those of other genera and species of lactic acid bacteria known to date. Another preferred alternative for determining the attachment of species to the genus Lactobacillus is the use of species-specfic PCR primers that target the 16S-23S rRNA spacer region. Another preferred alternative is RAPD-PCR (Niaatu et al. in Antonie van Leenwenhoek (79), 1-6, 2001) by virtue of that a strain specific DNA pattern is generated which allows to determine the affiliation of an identified microorganisms to the genus of Lactobacillus. Further techniques useful for determining the affiliation of a microorganism to the genus of Lactobacillus are restriction fragment length polymorphism (RFLP) (Giraffa et al., Int. J. Food Microbiol. 82 (2003), 163-172), fingerprinting of the repetitive elements (Gevers et al., FEMS Microbiol. Lett. 205 (2001) 31-36) or analysis of the fatty acid methyl ester (FAME) pattern of bacterial cells (Heyman et al., FEMS Microbiol. Lett. 181 (1991), 55-62). Alternatively, lactobacilli can be determined by lectin typing (Annuk et al., J. Med. Microbiol. 50 (2001), 1069-1074) or by analysis of their cell wall proteins (Gatti et al., Lett. Appl. Microbiol. 25 (1997), 345-348.

The above mentioned microorganisms are preferably lactic acid bacteria belonging to the genus of Lactobacillus, more preferably Lactobacillus species as described herein. Even more preferably said Lactobacillus is Lactobacillus paracasei or Lactobacillus rhamnosus. However, the Lactobacillus species are not limited thereto. As described above, the microorganisms used according to the present invention as described in the claims belong to the genus Lactobacillus. The above mentioned microorganisms may preferably be "isolated" or "purified". The term "isolated" means that the material is removed from its original environment, e.g. the natural environment if it is naturally occurring. For example, a naturally-occurring microorganism, preferably a Lactobacillus species, separated from some or all of the coexisting materials in the natural system, is isolated. Such a microorganism could be part of a composition, and is to be regarded as still being isolated in that the composition is not part of its natural environment.

The term "purified" does not require absolute purity; rather, it is intended as a relative definition. Individual microorganisms obtained from a library have been conventionally purified to microbiological homogeneity, i.e. they grow as single colonies when streaked out on agar plates by methods known in the art. Preferably, the agar plates that are used for this purpose are selective for Lactobacillus species. Such selective agar plates are known in the art.

More preferably, the above mentioned microorganism belonging to the group of lactic acid bacteria is selected from the group consisting of Lactobacillus paracasei or Lactobacillus rhamnosus having DSMZ accession number DSM 16667 (L paracasei ssp. paracasei Lb-Ob-K1), DSMZ accession number DSM 16668 (L. paracasei ssp. paracasei Lb-Ob-K2), DSMZ accession number DSM 16669 (L. paracasei ssp. paracasei Lb-Ob-K3), DSMZ accession number DSM 16670 (L. paracasei ssp. paracasei Lb-Ob-K4), DSMZ accession number DSM 16671 (L. paracasei ssp. paracasei Lb-Ob-K5), DSMZ accession number DSM 16672 (L. rhamnosus Lb-Ob-K6) and DSM accession number DSM 16673 (L. rhamnosus Lb-Ob-K7) or a mutant or derivative thereof, wherein said mutant or derivative retains the capability to specifically bind to mutans Streptococci. The term "Lactobacillus paracasei or Lactobacillus rhamnosus having DSMZ accession number" relates to cells of a microorganism belonging to the species Lactobacillus paracasei or Latobacillus rhamnosus deposited with the Deutsche Sammlung fur Mikroorganismen und Zellkulturen GmbH ("DSMZ") on August 26, 2004 and having the following deposit numbers DSM 16667, 16668, 16669, 16670, 16671, 16672 or 16673. The DSMZ is located at the Mascheroder Weg 1b, D-38124 Braunschweig, Germany. The aforementioned DSMZ deposits were made pursuant to the terms of the Budapest treaty on the international recognition of the deposit of microorganisms for purposes of patent procedure.

"A mutant or derivative" of the above mentioned microorganism belonging to the group of lactic acid bacteria, preferably of the deposited Lactobacillus paracasei or Lactobacillus rhamnosus cells has preferably the same characteristics as the respective deposited strains, i.e. it retains the capability to specifically bind to mutans Streptococci, preferably with the binding characteristics as described herein above. For example, said derivative can be genetically engineered. In the context of the present invention the term "genetically engineered" is used in its broadest sense for methods known to the person skilled in the art to modify desired nucleic acids in vitro and in vivo such that genetic modifications are affected and genes are altered by recombinant DNA technology. Accordingly, it is preferred that said methods comprise cloning, sequencing and transformation of recombinant nucleic acids. For this purpose appropriate vectors including expression vectors for Lactobacillus species as, for example, described in EP-B1 506 789, EP-B1 316 677, EP-B1 251 064, EP-B1 218 230, EP-B1 133 046 or WO 89/01970.

Primers, enzymes, further host cells for cloning of intermediate constructs and the like can be used and are known by the skilled artisan. Preferably, genetically engineered mutants comprise cells of the above mentioned microorganism belonging to the group of lactic acid bacteria, preferably of the deposited Lactobacillus species harbouring recombinant nucleic acids either comprised in their bacterial chromosome or on (a) plasmid(s) or comprised in their bacterial chromosome and/or (a) plasmid(s). Said recombinant nucleic acids are preferably foreign to the above mentioned microorganism belonging to the group of lactic acid bacteria. By "foreign" it is meant that the polynucleotide or nucleic acid molecule is either heterologous with respect to the host cell, this means derived from a cell or organism with a different genomic background, or is homologous with respect to the host cell but located in a different genomic environment than the naturally occurring counterpart of said nucleic acid molecule. This means that, if the nucleic acid molecule is homologous with respect to the host cell, it is not located in its natural location in the genome of said host cell, in particular it is surrounded by different genes. In this case the polynucleotide may be either under the control of its own promoter or under the control of a heterologous promoter. The above described vector or nucleic acid molecule, which is present in the host cell may either be integrated into the genome of the host cell or it may be maintained in some form extrachromosomally. In this respect, it is also to be understood that the above described nucleic acid molecule can be used to restore or create a mutant gene via homologous recombination. Plasmids may be low, medium or high copy number plasmids. Said genetically engineered mutants may harbour nucleic acids encoding a glucanase or mutanase which is capable of degrading the mutan specific 1,3-glycosidic bond of saccharose subunits. Fungal glucanases are, for example, described in Fuglsang et al., J. Biol. Chem. 275 (2000), 2009-2018. It is also envisaged that genetically engineered mutants comprise cells harbouring recombinant nucleic acids encoding antibodies which are preferably secreted or anchored in the bacterial cell wall. The term "antibody" encompasses intact antibodies as well as antibody fragments thereof, like, separated light and heavy chains, Fab, Fab/c, Fv, Fab', F(ab')2. The term "antibody" also comprises humanized antibodies, bifunctional antibodies and antibody constructs, like single chain Fvs (scFv) or antibody-fusion proteins. It is also envisaged in context of this invention that the term "antibody" comprises antibody constructs which may be expressed in cells of the derivative of the above mentioned deposited microorganism, e.g. antibody constructs which may be transformed via, inter alia, vectors by methods known in the art. It is in particular envisaged that such antibody constructs specifically recognize, for example, the streptococcal antigen I/II. Such an approach is, for example, described in Krueger et al., Nat. Biotechnol. 20 (2002). 702-706 or Shiroza, Biochim Biophys Acta 1626 (2003). 57-64.

Secretion of the expressed antibody is preferably achieved by operatively linking the nucleic acid encoding an antibody to a secretion signal sequence. Anchoring in the bacterial cell wall could be achieved by making use of the mechanism of the enzyme sortase. Namely, surface proteins of gram-positive bacteria are linked to the bacterial cell wall by a mechanism that involves cleavage of a conserved Leu-Pro-X-Thr-Gly (LPXTG) motif and that occurs during assembly of the peptidoglycan cell wall. Accordingly, the nucleic acid molecule encoding an antibody may be fused to a sequence encoding the aforementioned conserved motif, which is used by sortase to anchor proteins in the bacterial cell wall.

It is also envisaged that the above mentioned microorganism belonging to the group of lactic acid bacteria, preferably the deposited Lactobacillus species be genetically modified to harbor a nucleic acid molecule encoding reuterin which is an antimicrobial substance effective, inter alia, against Streptococcus mutans. Reuterin is, for example, described in Talarico et al., Chemother. 33 (1989), 674-679.

A mutant of the above mentioned microorganism belonging to the group of lactic acid bacteria, preferably a mutant of the deposited Lactobacillus strains is preferably artificially mutated. In accordance with the present invention, the term "mutated" means (a) permanent modification(s) of genetic material, i.e. nucleic acids, caused, for example, naturally or by physical means or chemical compounds/substances/agents, such as EMS or ENU. Said modifications include point mutations, like transitions or transversions, deletion/insertion/addition of one or more bases within a nucleic acid/gene/chromosome thereby modifying the nucleic acid/gene/chromosome which can cause, inter alia, aberrant gene expression/transcription/translation or inactive gene products, constitutive active/inactive gene products leading to e.g. dominant-negative effects. Preferably, a mutation leads to in increased capability of specifically binding mutans Streptococci. Thus, it is also preferred that the mutant cells of the deposited microorganism which harbour (a) mutation(s) in (a) desired gene(s) or in which (a) mutation(s) in (a) desired gene(s) is induced by methods known to the person skilled in the art. It is also known in the prior art that mutated or genetically engineered bacterial cells can be selected by any suitable method/phenotype. In the context of the present invention, a mutant having an increased capability to specifically bind to mutans Streptococci can be tested in accordance with the methods described in the appended Examples. The term "mutant", however, also includes cells of above mentioned microorganism belonging to the group of lactic acid bacteria, preferably cells of the deposited microorganism, which harbour naturally-occurring, spontaneous mutations in their genome, i.e. bacterial chromosome. "Spontaneous mutations" are mutations that arise naturally, i.e., without direct genetic manipulation by man, or by exposure to a mutagen. Selection of spontaneous mutant can be accomplished by culturing the strain and selecting the desired variants by, for example, the variant bacterium's capability to show an improved binding to mutans Streptococci. Methods for selection of spontaneous mutants are well known in the art (see, for example, Sambrook, Russell "Molecular Cloning, A Laboratory Manual", Cold Spring Harbor Laboratory, N.Y. (2001); Ausubel, "Current Protocols in Molecular Biology". Green Publishing Associates and Wiley Interscience, N.Y. (1989)). For example, such mutations may occur during cultivation, for example, during the normal cell division process coupled with DNA replication or during passaging and/or preserving the mutant of the above mentioned microorganism belonging to the group of lactic acid bacteria.

The oral cavity is home to many different species of streptococci and it is not surprising, considering they share the same habitat, that they have many features in common. Thus, it is preferable that the above mentioned microorganism belonging to the group of lactic acid bacteria binds specifically to mutans Streptococci. Accordingly, the term "specifically binding" in the context of the present invention means that the above mentioned microorganism belonging to the group of lactic acid bacteria, preferably a microorganism belonging to the genus of Lactobacillus binds to a mutans Streptococcus, in particular Streptococcus mutans, but does not bind to most other, preferably to no other species belonging to the genus Streptococcus, wherein other species belonging to the genus of Streptococcus are those described in Example 6. Namely, the above mentioned microorganism belonging to the group of lactic acid bacteria does preferably not bind to bacteria belonging to the species of Streptococcus salivarius, preferably belonging to the subspecies thermophilus, to the species Streptococcus oralis, to the species Streptococcus mitis and/or to the species Streptococcus sanguinis. More preferably, it does not bind to Streptococcus salivarius ssp. thermophilus (identified by API 50 CH (Biomerieux, France), Streptococcus oralis (DSMZ 20066), Streptococcus oralis (DSMZ 20395), Streptococcus oralis (DSMZ 20627), Streptococcus mitis (DSMZ 12643) and/or Streptococcus sanguinis (DSMZ 20567). In addition, said microorganism preferably does not bind to bacteria belonging to genera other than Streptococcus, e.g. belonging to the genus of Staphylococcus. More preferably, it does not bind to bacteria belonging to the species Staphylococcus epidermidis. Most preferably, it does not bind to Staphylococcus epidermidis (DSMZ 1798) and/or Staphylococcus epidermidis (DSMZ 20044).

As already mentioned above, it has surprisingly be found that lactic acid bacteria which are selected for their binding to Streptococcus mutans while not binding to the other Streptococcus species Streptococcus salivarius, Streptococcus oralis, Streptococcus mitis and/or Streptococcus sanguinis, show the capability to also bind other mutans Streptococci, i.e. other Streptococcus species which show the above mentioned common characteristics and which are also suspected as causative agents for caries. Thus, lactic acid bacteria which show the above-mentioned specific binding characteristics for Streptococcus mutans show the capacity to be also used in connection whith the prophylaxis or treatment of caries caused by mutans Streptococci other than Streptococcus mutans, in particular S. sobrinus. Thus, in a preferred embodiment the lactic acid bacteria are characterized in that they display the above described binding characteristics (i.e. (i) resistance to heat treatment; and (ii) resistance to protease treatment; and (iii) calcium dependency; and (iv) formation of the binding within a pH range between 4.5 and 8.5; and (v) formation of the binding in the presence of saliva) in relation to Streptococcus mutans and the binding is tested in an assay as described herein above in which Streptococcus mutans is used as a bacterium belonging to the group of mutans Streptococci.

For the test of specific binding, preferably each of the aforementioned oral bacteria are preferably mixed in a volumetric ratio of 3:1 with Lactobacillus cultures as described herein above and aggregation is assayed as described herein and for instance in Example 4 or Example 5, preferably as described in Example 5.

It was shown that the above mentioned Lactobacillus paracasei, preferably L. paracasei ssp. paracasei does not aggregate any of the aforementioned oral bacteria belonging to the genus of Streptococcus and it does not bind the bacteria belonging to the genus of Staphylococcus mentioned herein above. The above mentioned Lactobacillus rhamnosus strains were shown to not aggregate all of the above mentioned Streptococcus and Staphylococcus species, apart from Streptococcus salivarius ssp. thermophilus. Preferably the term "specifically binding" also means that the above mentioned microorganism belonging to the group of lactic acid bacteria binds to such mutans Streptococci strains which have the capability to be a cariogenic dental pathogen.

The specific binding reaction comprises binding and, preferably, aggregating mutans Streptococcus cells as described herein by the above mentioned microorganism belonging to the group of lactic acid bacteria in the mouth. This specific binding leads, in consequence, to flushing away the mutans Streptococcus cells by, for example, salivary flow or by a mouth rinse or mouth wash and the like as described herein. The mouth defines the oral cavity of mammals, preferably humans or animals such as pets, composed by the oral mucosa (gums, lips, cheeks, palate and floor of the mouth), the tongue and the teeth (including artficial structures). Preferably, the specific binding reaction of the above mentioned microorganism belonging to the group of lactic acid bacteriato mutans Streptococci prevents mutans Streptococcus cells from attaching to the surface of a tooth or teeth (or while not being bound by theory could lead to detachment of mutans Streptococcus cells from the surface of a tooth or teeth) In consequence, the specific binding reaction results in flushing away mutans Streptococcus cells out of the mouth, thereby diminishing the causative agent of caries and, thus, preventing and/or treating caries.

It is believed that the above mentioned microorganism belonging to the group of lactic acid bacteria may bind specifically to the streptococcal antigen I/II which is also known as antigen B, IF, P1, SR, MSL-1 or PAc. However, the above mentioned microorganism belonging to the group of lactic acid bacteria may bind to any other protein or surface structure of mutans Streptococci, thereby aggregating mutans Streptococci and flushing them out of the oral cavity as described herein. It is known that Streptococcus mutans binds via said streptococcal antigen I/II to the pellicle. Accordingly, when the above mentioned microorganism belonging to the group of lactic acid bacteria may bind, for example, to said streptococcal antigen I/II, Streptococcus mutans as well as the other microorgansims belonging to the group of mutans Streptococci are hampered to bind to the surface of teeth which thus helps to prevent and/or treat caries.

The pellicle is a clear, thin covering containing proteins and lipids (fats) found in saliva. It is formed within seconds after a tooth surface is cleaned. Pellicle formation is the first step in dental plaque formation. Dental plaque is a soft deposit that accumulates on the teeth. Plaque can be defined as a complex microbial community, with greater than 10¹⁰ bacteria per milligram. It has been estimated that as many as 400 distinct bacterial species may be found in plaque. In addition to the bacterial cells, plaque contains a small number of epithelial cells, leukocytes, and macrophages. The cells are contained within an extracellular matrix, which is formed from bacterial products and saliva. The extracellular matrix contains protein, polysaccharide and lipids. One of the proteins present in saliva is agglutinin which is on the one hand thought to lead to a partial removal of Streptococcus mutans from the mouth, however, is on the other hand suspected to facilitate adhesion of Streptococcus mutans to the surface of teeth, thereby facilitating the initial attachment of Streptococcus mutans to teeth and, thus, onset of caries.

Whether the above mentioned microorganism belonging to the group of lactic acid bacteria specifically binds to mutans Streptococci as defined herein above can easily be tested, inter alia, by comparing the reaction of said microorganism with mutans Streptococcus cells with a microorganism also belonging to the genus of Lactobacillus that, however, does not specifically bind to mutans Streptococci by preferably employing the method as described herein above and in the appended Examples herein below.

Preferably, the above mentioned microorganism belonging to the group of lactic acid bacteria is capable of specifically binding to Streptococcus mutans serotype c (DSMZ 20523) and/or serotype e (NCTC 10923) and/or serotype f (NCTC 11060) and/or Streptococcus sobrinus DSM 20742 and/or Streptococcus ratti DSM 20564 and/or Streptococcus cricetus DSM 20562 and/or Streptococcus ferus DSM 20646 and/or Streptococcus macacae DSM 20714.

This means that the above mentioned microorganism belonging to the group of lactic acid bacteria preferably binds to at least one microorganism selected from the group consisting of Streptococcus mutans serotype c (DSMZ 20523), serotype e (NCTC 10923), serotype f (NCTC 11060), Streptococcus sobrinus. DSM 20742, Streptococcus ratti DSM 20564, Streptococcus cricetus DSM 20562, Streptococcus ferus DSM 20646 and Streptococcus macacae DSM 20714. More preferably, the above mentioned microorganism belonging to the group of lactic acid bacteria binds to any combination, grouping of subgrouping of the above mentioned bacteria. Even more preferably, the above mentioned microorganism belonging to the group of lactic acid bacteria binds to all of the above mentioned bacteria. In accordance with the present invention a "serotype" is an antigenic property of a bacterial cell, preferably of a Streptococcus mutans or Streptococcus sobrinus cell identified by serological methods known in the art.

As described above, the specific binding of the above mentioned microorganism belonging to the group of lactic acid bacteria to mutans Streptococci is resistant to heat treatment. Accordingly, the above mentioned microorganism belonging to the group of lactic acid bacteriais treated with heat, for example, at a temperature above 15°C or 37°C. More preferably, the cells are incubated at a temperature of more than 55°C, even more preferably of more than 65°C, particularly preferred of more than 95°C and most preferred at 121°C. After cooling down, the capability of the above mentioned microorganism belonging to the group of lactic acid bacteriato specifically bind the mutans Streptococci is determined as described herein.

The corresponding temperature can depend on the specific Lactobacillus species but can be easily determined by the skilled person by routine experimentation, e.g. by incubating the corresponding cells at different temperatures and determining the amount of Lactobacillus cells which is still capable of specifically binding to mutans Streptococci by using methods as described in the examples herein.

Generally, the heat treatment should last for a period of time of at least 1 minute. Preferably, the heat treatment lasts for a period of time of at least n minutes, wherein n is an integer in the range of 2 to 60, with n=20 being particularly preferred, In connection with the use of the present invention as described in the claims, heat treatment is carried out at a temperature of more than 95°C for at least 20 minutes. However, there is in principle no upper limit for the time of incubation. However, it is preferably no longer than 4, 3, 2 or 1 hour(s). The most preferred heat treatment is at least 20 minutes at a temperature of 121°C in a saturated steam having an atmospheric pressure of 2 bar. The most preferred heat treatment is considered as abolishing any function of a protein and of any vitality of cells, which thus distinguishes the above mentioned microorganism belonging to the group of lactic acid bacteriafrom other microorganism in that it is still capable of the specfically binding to mutans Streptococci. Hence, it is very useful for use in any food, feed, drink or composition in the context of the present invention if it is desired that the microorganism should not be alike.

Preferably, above mentioned microorganism belonging to the group of lactic acid bacteria, which has been subjected to a heat treatment as described herein above, has an increased capability of specifically binding to a bacterium belonging to the group of mutans Streptococci. The term "increased capability" means an augmentation of the binding, as measurable, for example, by an assay as described herein above, preferably as described in Example 5, by at least 5%, preferably at least 10%, more preferably at least 20% and most preferably at least 30%. In a preferred embodiment, an above mentioned microorganism belonging to the group of lactic acid bacteria, which has an increased capability of specifically binding to a bacterium belonging to the group of mutans Streptococci upon having been subjected to a heat treatment belongs to the species Lactobacillus paracasei, more preferably to L. paracasei ssp. paracasei and most preferably to L. paracasei ssp. paracasei Lb-Ob-K5 (DSM 16671).

The specific binding of above mentioned microorganism belonging to the group of lactic acid bacteria, specifically of the genus Lactobacillus, is furthermore characterized by its resistance to protease treatment which is treatment with a protease selected from the group consisting of pronase E, proteinase K, trypsin and chymotrypsin. These proteinases are proteases, which show no specificity and, thus, are considered as degrading any protein being on the cell surface of a microorganism. Other proteases, which are known to have preferences for certain patterns of amino acid residues are elastase, thrombin, aminopeptidase I, carboxypeptidase, dostripain, endoproteinase, papain, pepsin or proteases. The latter proteases could also be used to test whether the specific binding of the above mentioned microorganism belonging to the group of lactic acid bacteriato mutans Streptococci is resistant to the latter more specific proteases. Thus, after protease treatment, which is described in the appended Examples, the above mentioned microorganism belonging to the group of lactic acid bacteria is still capable of specifically binding to mutans Streptococci.

In addition, the specific binding of the above mentioned microorganism belonging to the group of lactic acid bacteria is furthermore characterized by its dependency on calcium. Preferably, the specific binding takes place in the presence of a concentration of calcium ions between 0.05 mM and 500 mM, preferably between 1 mM and 100 mM. Particularly preferred the calcium concentration is between 2 mM and 30 mM. The dependency of the specific binding on calcium can be tested as described in the appended Examples.

Moreover, the specific binding to the above mentioned microorganism belonging to the group of lactic acid bacteria is maintained over a pH range between 4.0 and 9.0, preferably between 4.0 and 7.0. In particular, the pH value at which the specific binding takes still place is preferably 4.5. Assaying of the maintenance of the specific binding over the pH range described above is shown in the appended Examples. Furthermore, the specific binding is independent of magnesium. Thus, it is not necessary that magnesium ions or magnesium salts are present which is demonstrated in the appended Examples.

A still further characteristic of the specific binding is its occurrence in the presence of saliva. Saliva is an exogenous secrete which is synthesized by the salivary glands. It is a complex liquid containing, apart from about 99% water a multiplicity of organic and inorganic compounds. Physiological ingredients of saliva are, inter alia, enzymes, e.g., amylases, carboanhydrases, lysozyme, peroxidases or proteins, e.g., mucins, lactoferrin, proline-rich proteins, cystatines, histatines or statherines or soluble IgA. Thus, although a variety of potentially interfering substances are present in saliva, the specific binding of the above mentioned microorganism belonging to the group of lactic acid bacteria was not disturbed or hampered. For testing the specific binding in the presence of saliva, it is preferred that saliva is used which contains preferably the Streptococcus species described in Example 6 and/or the Staphylococcus species pf Example 6. If, however, Lactobacillus rhamnosus species as described above are tested for specific binding to mutans Streptococci in the presence of saliva, it is preferred that Streptococcus salivarius ssp. thermophilus is omitted. The specific binding is assayed as described herein.

The aforementioned characteristics of the above mentioned microorganism belonging to the group of lactic acid bacteria renders it to be a robust and effective agent for preventing and/or treating caries since it is mainly administered in various forms to the mouth including the oral cavity and teeth where, inter alia, saliva including certain proteases and low pH values after ingestion of carbohydrate containing food stuff is present. Moreover, the resistance to heat has beneficial effects in adding the above mentioned microorganism belonging to the group of lactic acid bacteria as additive to food stuff during the preparation of said food stuff. Namely, food stuff is often heat sterilized, pre-cooked, pasteurized and the like which is detrimental for viability of microorganisms.

In another aspect of the present invention a derivative of the above mentioned microorganism belonging to the group of lactic acid bacteria is employed in the described use. The term "derivative of the above mentioned microorganism belonging to the group of lactic acid bacteria" means an inactivated form or fragment of the above mentioned microorganism belonging to the group of lactic acid, bacteria, which is thermally inactivated or lyophilized, wherein said inactivated form or fragment retains the capability of specifically binding mutans Streptococci.

According to the present invention the term "inactivated form of the above mentioned microorganism belonging to the group of lactic acid bacteria" includes a dead or inactivated cell of the above mentioned microorganism belonging to the group of lactic acid bacteria, preferably of the Lactobacillus species disclosed herein which is no longer capable to form a single colony on a plate specific for microorganisms belonging to the genus of Lactobacillus. Said dead or inactivated cell may have either an intact or broken cell membrane. Methods for killing or inactivating cells of the above mentioned microorganism belonging to the group of lactic acid bacteria are known in the art. EI-Nezami et al., J. Food Prot. 61 (1998), 466-468 describes a method for inactivating Lactobacillus species by UV-irradiation. Preferably, the cells of the above mentioned microorganism belonging to the group of lactic acid bacteria are thermally inactivated or lyophilised as described in the appended Examples. Lyophilization of the cells as described above has the advantage that they can be easily stored and handled while retaining their capability to specifically bind to mutans Streptococci. Moreover, lyophilised cells can be grown again when applied under conditions known in the art to appropriate liquid or solid media. Lyophilization is done by methods known in the art. Preferably, it is carried out for at least 2 hours at room temperature, i.e. any temperature between 16°C and 25°C. Moreover, the lyophilized cells of the above mentioned microorganism belonging to the group of lactic acid bacteria are stable for at least 4 weeks at a temperature of 4°C so as to still specifically bind to mutans Streptococci as is shown in Example 7 or 8, herein below. Thermal inactivation can be achieved by incubating the cells of the above mentioned microorganism belonging to the group of lactic acid bacteria for at least 2 hours at a temperature of 170°C. Yet, thermal inactivation is preferably achieved by autoclaving said cells at a temperature of 121°C for at least 20 minutes in the presence of saturated steam at an atmospheric pressure of 2 bar. In the alternative, thermal inactivation of the cells of the above mentioned microorganism belonging to the group of lactic acid bacteria is achieved by freezing said cells for at least 4 weeks, 3 weeks, 2 weeks, 1 week, 12 hours, 6 hours, 2 hours or 1 hour at -20°C. It is preferred that at least 70%, 75% or 80%, more preferably 85%, 90% or 95% and particularly preferred at least 97%, 98%, 99% and more particularly preferred, 99.1%, 99.2%, 99.3%, 99.4%, 99.5%, 99.6%, 99.7%, 99.8% or 99.9% and most particularly preferred 100% of the cells of the analog of the above mentioned microorganism belonging to the group of lactic acid bacteria are dead or inactivated, however, they have still the capability to specifically bind to mutans Streptococci. Whether the inactivated form, analog or fragment of the above mentioned microorganism belonging to the group of lactic acid bacteria is indeed dead or inactivated can be tested by methods known in the art, for example, by a test for viability.

The term "inactivated form of the above mentioned microorganism belonging to the group of lactic acid bacteria" also describes lysates, fractions or extracts of the above mentioned microorganism belonging to the group of lactic acid bacteria, preferably of the Lactobacillus species disclosed herein, wherein said lysates, fractions or extracts are preferably capable of specifically binding to a bacterium belonging to the group of mutans Streptococci. This binding capability can be tested as described herein and in particular as described in the appended Examples. In case, a lysate, fraction or extract of a microorganism as described herein above, may not specifically bind to a bacterium belonging to the group of mutans Streptococci, then the skilled person can, for example, further purify said lysate, fraction or extract by methods known in the art, which are exemplified herein below, so as to remove substances which inhibit the binding. Afterwards the person skilled in the art can again test said lysate, fraction or extract whether it specifically binds to a bacterium belonging to the group of mutans Streptococci.

The term "lysate" means a solution or suspension in an aqueous medium of cells of the above mentioned microorganism belonging to the group of lactic acid bacteria that are broken. However, the term should not be construed in any limiting way. The cell lysate comprises, e.g., macromolecules, like DNA, RNA, proteins, peptides, carbohydrates, lipids and the like and/or micromolecules, like amino acids, sugars, lipid acids and the like, or fractions of it. Additionally, said lysate comprises cell debris which may be of smooth or granular structure. Preferably, said lysate comprises the cell wall or the cell membrane or both or portions or fragments of the cell wall or the cell membrane or of both. Methods for preparing cell lysates of microorganism are known in the art, for example, by employing French press, cells mill using glass or iron beads or enzymatic cell lysis and the like. In addition, lysing cells relates to various methods known in the art for opening/destroying cells. The method for lysing a cell is not important and any method that can achieve lysis of the cells of the above mentioned microorganism belonging to the group of lactic acid bacteria may be employed. An appropriate one can be chosen by the person skilled in the art, e.g. opening/destruction of cells can be done enzymatically, chemically or physically. Non-limiting examples for enzymes and enzyme cocktails are proteases, like proteinase K, lipases or glycosidases; non-limiting examples for chemicals are ionophores, detergents, like sodium dodecyl sulfate, acids or bases; and non-limiting examples of physical means are high pressure, like French-pressing, osmolarity, temperature, like heat or cold. Additionally, a method employing an appropriate combination of an enzyme other than the proteolytic enzyme, an acid, a base and the like may also be utilized. For example, the cells of the above mentioned microorganism belonging to the group of lactic acid bacteria are lysed by freezing and thawing, more preferably freezing at temperatures below -70°C and thawing at temperatures of more than 30°C, particularly freezing is preferred at temperatures below -75°C and thawing is preferred at temperatures of more than 35°C and most preferred are temperatures for freezing below -80°C and temperatures for thawing of more than 37°C. It is also preferred that said freezing/thawing is repeated for at least 1 time, more preferably for at least 2 times, even more preferred for at least 3 times, particularly preferred for at least 4 times and most preferred for at least 5 times.

Accordingly, those skilled in the art can prepare the desired lysates by referring to the above general explanations, and appropriately modifying or altering those methods, if necessary. Preferably, the aqueous medium used for the lysates as described is water, physiological saline, or a buffer solution. An advantage of a bacterial cell lysate is that it can be easily produced and stored cost efficiently since less technical facilities are needed.

Preferably, the term "extract" means a subcellular component of the above mentioned microorganism belonging to the group of lactic acid bacteria, e.g., a macromolecule, like a protein, DNA, RNA, a peptide, a carbohydrate, a lipid and the like and/or a micromolecule, like an amino acid, a sugar, a lipid acid and the like or any other organic compound or molecule, or a combination of said macromolecules and/or micromolecules or any fraction of it, wherein said extract retains the capability of specifically binding to a bacterium belonging to the group of mutans Streptococci. This specific binding can be tested as described herein and in particular as described in the appended Examples. Preferably, said extract comprises the cell wall or the cell membrane or both or portions or fragments of the cell wall or the cell membrane or of both. More preferably, the term "extract" refers to any of the above described subcellular components in a cell-free medium.

An extract may be obtained by lysing cells according to various methods known in the art for opening/destroying cells, as described herein above and/or as supernatant of a centrifugation procedure of a culture of the above mentioned microorganism belonging to the group of lactic acid bacteria in any appropriate liquid, medium or buffer known to the person skilled in the art or of a lysate of such a culture or any other suitable cell suspension. More preferably, the extract may be a purified lysate or cell culture supernatant or any fraction or subportion thereof, wherein said purified lysate or cell culture supernatant or any fraction or subportion thereof retains the capability of specifically binding to a bacterium belonging to the group of mutans Streptococci. This binding can be tested as described herein and in particular as described in the appended Examples. Suitable methods for fractionation and purification of a lysate, culture supernatant or an extract are known to the person skilled in the art and comprise, for example, affinity chromatography, ion-exchange chromatography, size-exclusion chromatography, reversed phase-chromatography, and chromatography with other chromatographic material in column or batch methods, other fractionation methods, e.g., filtration methods, e.g., ultrafiltration, dialysis, dialysis and concentration with size-exclusion in centrifugation, centrifugation in density-gradients or step matrices, precipitation, e.g., affinity precipitations, salting-in or salting-out (ammoniumsulfate-precipitation), alcoholic precipitations or any other suitable proteinchemical, molecular biological, biochemical, immunological, chemical or physical method.

Lysates are also preparations of fractions of molecules from the above-mentioned lysates. These fractions can be obtained by methods known to those skilled in the art, e.g., chromatography, including, e.g., affinity chromatography, ion-exchange chromatography, size-exclusion chromatography, reversed phase-chromatography, and chromatography with other chromatographic material in column or batch methods, other fractionation methods, e.g., filtration methods, e.g., ultrafiltration, dialysis, dialysis and concentration with size-exclusion in centrifugation, centrifugation in density-gradients or step matrices, precipitation, e.g., affinity precipitations, salting-in or salting-out (ammoniumsulfate-precipitation), alcoholic precipitations or other proteinchemical, molecular biological, biochemical, immunological, chemical or physical methods to separate above components of the lysates. In a preferred embodiment those fractions, which are more immunogenic than others are preferred. Those skilled in the art are able to choose a suitable method and determine its immunogenic potential by referring to the above general explanations and specific explanations in the examples herein, and appropriately modifying or altering those methods, if necessary.

Accordingly, the term "inactivated form of the above mentioned microorganism belonging to the group of lactic acid bacteria" als describes filtrates of a microorganism as described herein above, preferably of the Lactobacillus species disclosed herein, wherein said filtrates preferably retain the capability of specifically binding to a bacterium belonging to the group of mutans Streptococci. This binding can be tested as described herein and in particular as described in the appended Examples. In case, a filtrate of the above mentioned microorganism belonging to the group of lactic acid bacteria, as described herein above, may not specifically bind to a bacterium belonging to the group of mutans Streptococci, then the skilled person can, for example, further purify said filtrate by methods known in the art, which are exemplified herein below, so as to remove substances which inhibit the binding. Afterwards the person skilled in the art can again test said filtrate whether it specifically binds to a bacterium belonging to the group of mutans Streptococci.

The term "filtrate" means a cell-free solution or suspension of a microorganism as described herein above which has been obtained as supernatant of a centrifugation procedure of a culture of the above mentioned microorganism belonging to the group of lactic acid bacteria in any appropriate liquid, medium or buffer known to the person skilled in the art. However, the term should not be construed in any limiting way. The filtrate comprises, e.g., macromolecules, like DNA, RNA, proteins, peptides, carbohydrates, lipids and the like and/or micromolecules, like amino acids, sugars, lipid acids and the like, or fractions of it. Methods for preparing filtrates of microorganisms are known in the art. In addition, "filtrate" relates to various methods known in the art. The exact method is not important and any method that can achieve filtration of the cells of the microorganism of the invention, as described herein above, may be employed.

"A fragment of the above mentioned microorganism belonging to the group of lactic acid bacteria" describes any part of the cells of the above mentioned microorganism belonging to the group of lactic acid bacteria. According to the invention said fragment is a membrane fraction obtained by a membrane-preparation and retains the capability of specifically binding to the group of mutants Streptococci. Membrane preparations of microorganisms belonging to the genus of Lactobatillus can be obtained by methods known in the art, for example, by employing the method described in Rollan et al., Int. J. Food Microbiol. 70 (2001), 303-307, Matsuguchi et al., Clin. Diagn. Lab. Immunol. 10 (2003), 259-266 or Stentz et al., Appl. Environ. Microbiol. 66 (2000), 4272-4278 or Varmanen et al., J. Bacteriology 182 (2000), 146-154. Alternatively, a whole cell preparation is also envisaged. Preferably, the herein described derivative or fragment of the above mentioned microorganism belonging to the group of lactic acid bacteria retains the capability of specifically binding to mutans Streptococci which is described in detail herein.

As defined in the claims, in case a derivative is used according to the present invention, said derivative of the microorganism is an inactivated form of said microorganism or a fragment of said microorganism, said microorganism being thermally inactivated or lyophilized, wherein said fragment is a membrane fraction obtained by a membrane preparation and wherein said inactivated form or said fragment retains the capability of specifically binding a bacterium belonging to the group of mutans Streptococci.

One aspect in connection with the present invention relates to the use of the above mentioned microorganism belonging to the group of lactic acid bacteria, a derivative or mutant or an analog or fragment thereof for the preparation of an anticariogenic composition, preferably a pharmaceutical or cosmetic composition, for the treatment or prevention of caries caused by mutans Streptococci other than Streptococcus mutans. Preferably, the composition comprises a microorganism belonging to the group of lactic acid bacteria, which is capable of specifically binding to mutans Streptococci or a mutant, derivative or fragment of this microorganism. More preferably, this microorganism is a deposited microorganism as described herein above or a mutant or derivative thereof or an analog or fragment of said microorganism. In a further preferred embodiment, said composition comprises a microorganism as described above in an amount between 10² to 10¹² cells, preferably 103 to 10⁸ cells per mg in a solid form of the composition. In case of a liquid form of compositions, the amount of the microorganisms is between 10² to 1013 cells per ml. However, for specific compositions the amount of the microorganism may be different as is described herein. A preferred anticariogenic composition of the present invention does not contain lactose in a range between 1% (w/w) and 6% (w/w). It is also preferred that the composition contains not more than 1%(w/w) lactose, e.g. it contains less than 1%, preferably less than 0.9% (w/w), 0.8% (w/w) lactose, etc. or that the composition contains more than 6%, 7%, 8% etc. (w/w) lactose. Alternatively, but also preferred is that the composition does not contain lactose.

In a further aspect in connection with the present invention, such an anticariogenic composition may be produced by comprising the steps of formulating a microorganism belonging to the group of lactic acid bacteria which is capable of specifically binding to mutans Streptococci or a mutant, derivative, analog or fragment of this microorganism with a cosmetically, orally or pharmaceutical acceptable carrier or excipient. Preferably, this microorganism is a deposited microorganism as described herein above or a mutant, derivative or fragment thereof. A preferred anticariogenic composition as used in accordance with the present invention does not contain lactose in a range between 1% (w/w) and 6% (w/w). It is also preferred that the composition contains not more than 1%(w/w) lactose, e.g. it contains less than 1%, preferably less than 0.9% (w/w), 0.8% (w/w) lactose, etc. or that the anticariogenic composition contains more than 6%, 7%, 8% etc. (w/w) lactose. Alternatively, but also preferred is that the anticariogenic composition does not contain lactose.

The term "composition", as used in accordance with the present invention, relates to (a) composition(s), which comprise(s) at least one microorganism or mutant or derivative as described above, preferably a deposited microorganism as described above or an analog or fragment of said microorganism. It is envisaged that the compositions as used in accordance with the present invention, which are described herein below comprise the aforementioned ingredients in any combination. It may, optionally, comprise at least one further ingredient suitable for preventing and/or treating caries. Accordingly, it may optionally comprise any combination of the hereinafter described further ingredients. The term "ingredients suitable for preventing and/or treating caries" encompasses compounds or compositions and/or combinations thereof which either inhibit the binding of mutans Streptococci to the surface of teeth, to pellicles and/or which inactivate mutans Streptococci. More preferably, said term encompasses compounds or compositions and/or combinations thereof which may inhibit the adhesion of mutans Streptococci to the surface of teeth, inhibit the activity of glycosyltransferases of mutans Streptococci, inhibit or inactivate mutans Streptococci, inhibit the agglutinin-dependent binding of mutans Streptococci and/or inhibit the saccharose-dependent binding of mutans Streptococci as will be described below.

In particular, it is envisaged that the composition optionally further comprises compounds which inhibit the adhesion of mutans Streptococci to the tooth surface. Accordingly, it is envisaged that such a compound is an inhibitor of the competence signal peptide (CSP) of Streptococcus mutans. Said inhibitor is described in CA 2,302,861 as being a derivative or fragment of said CSP, which competitively inhibits binding of said CSP to its natural receptor, a histidine kinase receptor, or which is an antibody against said CSP. Said inhibitor prevents the development of a biofilm environment of dental plaque on the surface of teeth and, thus, prevents binding of mutans Streptococci. Alternatively, the composition as used in accordance with the present invention may optionally further comprise polypeptide fragments of the Streptococcus mutans I/II antigen that are useful in treating and/or preventing dental caries. Such polypeptide fragments are described in US 6,500,433. Namely, said polypeptide fragments may have the ability to adhere to the mammalian tooth surface by binding to agglutinin in a competitive manner with naturally occurring Streptococcus mutans antigen I/II, thus preventing or diminishing the adhesion of S. mutans to the tooth. Some of the peptides of US 6,500,433 have been shown to inhibit adhesion of S. mutans to a tooth surface model (whole human saliva adsorbed to the wells of polystyrene microtitre plates or hydroxyapatite beads). Accordingly, US 6,500,433 describes these peptides to comprise one or more adhesion sites and will adhere to a mammalian tooth in a competitive manner with naturally occurring SA I/II. Another optional ingredient of the composition as used in accordance with the present invention is the fimbrial-associated adhesion protein from Streptococus mutans, SmaA, or a fragment thereof as described in WO 00/66616. The SmaA protein is an adhesion protein from fimbriae of S. mutans which mediates attachment of the bacteria to the salivary pellicle, believed to be via binding to the 52 kd salivary protein, amylase. The mature SmaA protein has a molecular weight of about 65 kilodaltons (kd) as measured on a reducing polyacrylamide gel, exhibits the ability to bind amylase, and is the major immunodominant fimbrial protein of S. mutans. Accordingy, SmaA is believed to compete with mutans Streptococci for adhesion sites on the surface of teeth.

As described above, it is envisaged that compounds which inhibit mutans Streptococci glycosyltransferase activity are optionally further comprised in a composition as used in accordance with the present invention. For example, US 2004/0057908 describes a mixture of terpenoids and flavonoids which inhibit the activity of said glycosyltransferases. Duarte et al., Biol. Pharm. Bull. 26 (2003), 527-531 describe a novel type of propolis and its chemical fractions on glycosyltransferases and on growth and adherence of Streptococcus mutans. Accordingly, said novel type of propolis and its chemical fractions are contemplated to be an optional further ingredient of the composition as used in accordance with the present invention. Koo et al., J. Antimicrob. Chemother. 52 (2003), 782-789 describe that apigenin and tt-famesol inhibit Streptococcus mutans biofilm accumulation and polysaccharide production. Hence, apigenein and tt-farnesol are contemplated to be optionally comprised in the composition as used in accordance with the present invention. Since carbohydrate fatty acid esters are described in Devulapalle et al., Carbohydr. Res. 339 (2004), 1029-1034 to effect glycosyltransferase activity, said carbohydrate fatty acid esters are contemplated to be optionally comprised in the composition as used in accordance with the present invention.

Direct inhibition of Streptococcus mutans is, for example, described in WO 2004/000222. Namely, genetically modified bacteriophages specific for Streptococcus mutans are used for treating bacterial caries caused by Streptococcus mutans. WO 2004/017988 describes a composition of biologically active protease and at least one biologically active glycosidase which is used for treating bacterial caries. Imazato et al., Biomaterials 24 (2003), 3605-3609 describes that methacryloyloxydodecylpyridinium bromide (MDPB) is useful for inhibiting growth of Streptococcus mutans. Accordingly, it is envisaged that the aforementioned compounds may optionally be further comprised in the composition as used in accordance with the present invention.

Bovine milk lactoferrin described by Mitoma et al., J. Biol. Chem. 276 (2001), 18060-18065 or extracts of Helichrysum italicum described by Nostro et al., Lett. Appl. Microbiol. 38 (2004), 423-427 which inhibit agglubnin-dependent or saccharose-dependent binding of Streptococcus mutans are contemplated to be optionally further comprised in the composition as used in accordance with the present invention. Moreover, the composition as used in accordance with the present invention may optionally further comprise a mutanase (1,3-glucanase) which is, for example, described in DE 2152620 or Fuglsang (2000), loc. cit. or an antibiotic against Streptococcus mutans, for example, those described in US 6,342,385; US 5,932,469; US 5,872,001 or US 5,833,958. In addition, it is noted that the composition as used in accordance with the present invention may optionally comprise one or more of the aforementioned optional ingredients which are suitable for preventing and/or treating caries. Thus, said composition may contain at least two, three, four, five, etc., i.e. "n" optional ingredients, wherein "n" is an integer greater than 2 which is not limited. Said optional ingredients may be combined in any possible combination.

The composition may be in solid, liquid or gaseous form and may be, inter alia, in the form of (a) powder(s), (a) tablet(s), (a) film preparation(s), (a) solution(s) (an) aerosol(s), granules, pills, suspensions, emulsions, capsules, syrups, liquids, elixirs, extracts, tincture or fluid extracts or in a form which is particularly suitable for oral administration.

Liquid preparations suitable for oral administration, for example syrups can be prepared, using water, conventional saccharides such as sucrose, sorbitol and fructose, glycols such as polyethylene glycol and propylene glycol, oils such as sesame seed oil, olive oil and soybean oil, antiseptics such as p-hydroxybenzoate ester, preservatives such as p-hydroxybenzoate derivatives, for example p-hydroxybenzoate methyl and sodium benzoate, and other materials such as flavors, for example strawberry flavor or peppermint.

Further, preparations suitable for oral administration, for example tablets, powders and granules can be produced, using conventional saccharides such as sucrose, glucose, mannitol, and sorbitol, starch such as potato, wheat and corn, inorganic materials such as calcium carbonate, calcium sulfate, sodium hydrogen carbonate, and sodium chloride, plant powders such as crystal cellulose, licorice powder and gentian powder, excipients such as pinedex, disintegrators such as starch, agar, gelatin powder, crystal cellulose, carmellose sodium, carmellose calcium, calcium carbonate, sodium hydrogen carbonate and sodium alginate, lubricants such as magnesium stearate, talc, hydrogenated vegetable oils, macrogol, and silicone oil, binders such as polyvinyl alcohol, hydroxypropyl cellulose, methyl cellulose, ethyl cellulose, carmellose, gelatin, and starch glue fluid, surfactants such as fatty acid ester, and plasficizers such as glycerin. A film preparation(s) can be prepared by methods known in the art. An example for the preparation of a film is given in Example 27 herein.

In case of ordinary oral administration, the dose of the above described microorganism or analog or fragment could be (in dry weight) as described hereinabove with respect to the cell number or with respect to the mass, for example, 1 µg to 50 g, 1 µg to 10 g, 1 µg to 5 mg, 1 µg to 1 mg or any other weight per subject per day or in several portions daily. In case of dosing to non-human animals, further, the dose varies depending on the age and species of an animal and the nature or severity of the symptom thereof. Without any specific limitation, the dose for animals is 0.1 mg to 10 g per 1 kg body weight, preferably 1 mg to 1 g per 1 kg body weight once daily or in several portions daily. However, these doses and the number of dosages vary depending on the individual conditions.

Preferably, the anticariogenic composition as used in accordance with the present invention is a cosmetic composition further comprising a cosmetically acceptable carrier or excipient. More preferably, said cosmetic composition is a dentifrice, chewing gum, lozenge, mouth wash, mouse rinse, dental floss or dental tape which has an activity against mutans Streptococci. A preferred cosmetic composition as used in accordance with the present invention does not contain lactose in a range between 1% (w/w) and 6% (w/w). It is also preferred that the cosmetic composition contains not more than 1%(w/w) lactose, e.g. it contains less than 1 %, preferably less than 0.9% (w/w), 0.8% (w/w) lactose, etc. or that the cosmetic composition contains more than 6%, 7%, 8% etc. (w/w) lactose. Alternatively, but also preferred is that the cosmetic composition does not contain lactose.

The cosmetic composition as used in accordance with the present invention comprises the microorganism, mutant, derivative, analog or fragment thereof as described above in connection with the composition of the invention and further a cosmetically or orally acceptable carrier. Preferably, as mentioned in connection with the composition as used in accordance with the present invention the microorganism, mutant, derivative, analog or fragment thereof is a microorganism, mutant, derivative or fragment as described herein above. Preferably the cosmetic composition as used in accordance with the present invention is for use in oral applications. Accordingly, it may be in the form of a toothpaste, dentifrice, tooth powder, topical oral gel, mouth rinse, denture product, mouthspray, lozenge, oral tablet, chewing gum, dental floss or dental tape.

The term "orally or cosmetically acceptable carrier" as used herein means a suitable vehicle, which can be used to apply the present compositions to the oral cavity in a safe and effective manner. Such vehicle may include materials such as fluoride ion sources, additional anticalculus agents, buffers, other abrasive materials, peroxide sources, alkali metal bicarbonate salts, thickening materials, humectants, water, surfactants, titanium dioxide, flavor system, sweetening agents, xylitol, coloring agents, and mixtures thereof. The term "safe and effective amount" as used herein, means a sufficient amount to clean teeth and reduce stain/plaque/gingivitis/calculus without harming the tissues and structures of the oral cavity.

The pH of the present herein described compositions ranges preferably from about 3.0 to about 9.0, with the preferred pH being from about 5.5 to about 9.0 and the most preferred pH being 7.0 to about 8.5 or 9.0.

The cosmetic composition is a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral composition may be a single phase oral composition or may be a combination of two or more oral compositions.

The term "dentifrice", as used herein, means paste, gel, or liquid formulations unless otherwise specified. The dentifrice composition may be in any desired form, such as deep striped, surface striped, multilayered, having the gel surrounding the paste, or any combination thereof. The dentifrice composition may be contained in a physically separated compartment of a dispenser and dispensed side-by-side. Dentifrice compositions are, for example, described in EP-B1 0 617 608.

Preferred dentifrice compositions are described in Examples 21 to 24. In addition to the above described components, the dentifrice compositions of this invention can contain a variety of optional dentifrice ingredients some of which are described below. Optional ingredients include, for example, but are not limited to, adhesives, sudsing agents, flavouring agents, sweetening agents, additional antiplaque agents, additional abrasives, and colouring agents. These and other optional components are further described, for example, in US 5,004,597; US 4,885,155; US 3,959,458; and US 3,937,807.

For example, the toothpaste may include surfactants, chelating agents, fluoride sources, teeth whitening actives and teeth color modifying substances, thickening agents, humectants, flavouring and sweetening agents, alkali metal bicarbonate salt, miscellaneous carriers and/or other active agents.

One of the preferred optional agents as used in accordance with the present invention is a surfactant, preferably one selected from the group consisting of sarcosinate surfactants, isethionate surfactants and taurate surfactants. Preferred for use herein are alkali metal or ammonium salts of these surfactants. Most preferred herein are the sodium and potassium salts of the following: lauroyl sarcosinate, myristoyl sarcosinate, palmitoyl sarcosinate, stearoyl sarcosinate and oleoyl sarcosinate.

Another preferred optional agent is a chelating agent such as tartaric acid and pharmaceutically-acceptable salts thereof, citric acid and alkali metal citrates and mixtures thereof. Chelating agents are able to complex calcium found in the cell walls of the bacteria. Chelating agents can also disrupt plaque by removing calcium from the calcium bridges, which help hold this biomass intact

It is common to have an additional water-soluble fluoride compound present in dentifrices and other oral compositions in an amount sufficient to give a fluoride ion concentration in the composition at 25°C, and/or when it is used of from about 0.0025% to about 5.0% by weight, preferably from about 0.005% to about 2.0% by weight, to provide additional anticaries effectiveness. A wide variety of fluoride ion-yielding materials can be employed as sources of soluble fluoride in the present compositions. Examples of suitable fluoride ion-yielding materials are found in US 3,535,421 and US 3,678,154. Representative fluoride ion sources include stannous fluoride, sodium fluoride, potassium fluoride, sodium monofluorophosphate and many others. Stannous fluoride and sodium fluoride are particularly preferred, as well as mixtures thereof.

The oral care compositions as used in accordance with the present invention may also comprise teeth whitening actives, including bleaching or oxidizing agents such as peroxides, perborates, percarbonates, peroxyacids, persulfates, metal chlorites, and combinations thereof. Suitable peroxide compounds include hydrogen peroxide, urea peroxide, calcium peroxide, and mixtures thereof. A preferred percarbonate is sodium percarbonate. Other suitable whitening agents include potassium, ammonium, sodium and lithium persulfates and perborate mono- and tetrahydrates, and sodium pyrophosphate peroxyhydrate. Suitable metal chlorites include calcium chlorite, barium chlorite, magnesium chlorite, lithium chlorite, sodium chlorite, and potassium chlorite. The preferred chlorite is sodium chlorite. Additional whitening actives may be hypochlorite and chlorine dioxide.

In addition to bleaching agents as teeth whitening agents, teeth color modifying substances may be considered among the oral care actives useful in the present invention. These substances are suitable for modifying the color of the teeth to satisfy the consumer. These substances comprise particles that when applied on the tooth surface modify that surface in terms of absorption and, or reflection of light. Such particles provide an appearance benefit when a film containing such particles is applied over the surfaces of a tooth or teeth.

In preparing toothpaste or gels, it is necessary to add some thickening material to provide a desirable consistency of the composition, to provide desirable active release characteristics upon use, to provide shelf stability, and to provide stability of the composition, etc. Preferred thickening agents are carboxyvinyl polymers, carrageenan, hydroxyethyl cellulose, laponite and water soluble salts of cellulose ethers such as sodium carboxymethylcellulose and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture.

Another optional component of the topical, oral carriers of the compositions of the subject invention is a humectant. The humectant serves to keep toothpaste compositions from hardening upon exposure to air, to give compositions a moist feel to the mouth, and, for particular humectants, to impart desirable sweetness of flavor to toothpaste compositions. The humectant, on a pure humectant basis, generally comprises from about 0% to about 70%, preferably from about 5% to about 25%, by weight of the compositions herein. Suitable humectants for use in compositions of the subject invention include edible polyhydric alcohols such as glycerin, sorbitol, xylitol, butylene glycol, polyethylene glycol, and propylene glycol, especially sorbitol and glycerin.

Flavoring and sweetening agents can also be added to the compositions. Suitable flavoring agents include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof. Flavouring agents are generally used in the compositions at levels of from about 0.001% to about 5%, by weight of the composition.

Sweetening agents which can be used include sucrose, glucose, saccharin, dextrose, levulose, lactose as described herein above, mannitol, sorbitol, fructose, maltose, xylitol, saccharin salts, thaumatin, aspartame, D-tryptophane, dihydrochalcones, acesulfame and cyclamate salts, especially sodium cyclamate and sodium saccharin, and mixtures thereof. A composition preferably contains from about 0. 1 % to about 10% of these agents, preferably from about 0.1 % to about 1%, by weight of the composition.

The present invention may also include an alkali metal bicarbonate salt. Alkali metal bicarbonate salts are soluble in water and unless stabilized, tend to release carbon dioxide in an aqueous system. Sodium bicarbonate, also known as baking soda, is the preferred alkali metal bicarbonate salt. The present composition may contain from about 0.5% to about 30%, preferably from about 0.5% to about 15%, and most preferably from about 0.5% to about 5% of an alkali metal bicarbonate salt.

Water employed in the preparation of commercially suitable oral compositions should preferably be of low ion content and free of organic impurities. Water generally comprises from about 10% to about 50%, and preferably from about 20% to about 40%, by weight of the aqueous toothpaste compositions herein. These amounts of water include the free water which is added plus that which is introduced with other materials, such as with sorbitol. Titanium dioxide may also be added to the present composition. Titanium dioxide is a white powder, which adds opacity to the compositions. Titanium dioxide generally comprises from about 0.25% to about 5% by weight of the dentifrice compositions.

The pH of the present compositions is preferably adjusted through the use of buffering agents. Buffering agents, as used herein, refer to agents that can be used to adjust the pH of the compositions to a range of about 4.5 to about 9.5. Buffering agents include monosodium phosphate, trisodium phosphate, sodium hydroxide, sodium carbonate, sodium acid pyrophosphate, citric acid, and sodium citrate. Buffering agents can be administered at a level of from about 0.5% to about 10%, by weight of the present compositions. The pH of dentifrice compositions is measured from a 3:1 aqueous slurry of dentifrice, e.g., 3 parts water to 1 part toothpaste.

Other optional agents that may be used in the present compositions include dimethicone copolyols selected from alkyl- and alkoxy-dimethicone copolyols, such as C12 to C20 alkyl dimethicone copolyols and mixtures thereof. Highly preferred is cetyl dimethicone copolyol marketed under the Trade Name Abil EM90. The dimethicone copolyol is generally present in a level of from about 0.01% to about 25%, preferably from about 0.1% to about 5%, more preferably from about 0.5% to about 1.5% by weight. The dimethicone copolyols aid in providing positive tooth feel benefits. Other useful carriers include biphasic dentifrice formulations such as those disclosed in US 5,213,790; US 5,145,666; US 5,281,410; US 4,849,213 and US 4,528,180.

The present cosmetic compositions may also include other active agents, such as antimicrobial agents. Included among such agents are water insoluble non-cationic antimicrobial agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides. The water soluble antimicrobials include quaternary ammonium salts and bis-biquanide salts, among others. Triclosan monophosphate is an additional water soluble antimicrobial agent. The quaternary ammonium agents include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethylstearyl ammonium chloride, cetyl pyridinium chloride, quatemized 5-amino-1,3-bis(2-ethyl-hexyl)-5-methyl hexa hydropyrimidine, benzalkonium chloride, benzethonium chloride and methyl benzethonium chloride are exemplary of typical quaternary ammonium antibacterial agents. Other compounds are bis[4-(R-amino)-1-pyridinium] alkanes as disclosed in US 4,206,215. Other antimicrobials such as copper bisglycinate, copper glysinate, zinc citrate, and zinc lactate may also be included. Enzymes are another type of active that may be used in the present compositions. Useful enzymes include those that belong to the category of proteases, lytic enzymes, plaque matrix inhibitors and oxidases: Proteases include papain, pepsin, trypsin, ficin, bromelin; cell wall lytic enzymes include lysozyme; plaque matrix inhibitors include dextranses, mutanases; and oxidases include glucose oxidase, lactate oxidase, galactose oxidase, uric acid oxidase, peroxidases including horse radish peroxidase, myeloperoxidase, lactoperoxidase, chloroperoxidase. The oxidases also have whitening/cleaning activity, in addition to anti-microbial properties. Such agents are disclosed in US 2,946,725 and in US 4,051,234. Other antimicrobial agents include chlorhexidine, triclosan, triclosan monophosphate, and flavor oils such as thymol. Triclosan and other agents of this type are disclosed in US 5,015,466 and US 4,894,220. These agents, which provide anti-plaque benefits, may be present at levels of from about 0.01% to about 5.0%, by weight of the dentifrice composition.

The term "chewing gum" as defined herein means a confectionery composition which is suitable for chewing and which comprises any suitable amount of elastomer, known to the person skilled in the art, preferably an amount of 2% or greater, by weight of the composition. Suitable lozenge and chewing gum components are, for example, disclosed in US 4,083,955; US 6,770,264 or US 6,270,781. Preferred lozenges are those described in Examples 19 and 20. A preferred chewing gum composition is described in Example 25.

Compositions as used in accordance with the present invention preferably comprise an elastomer, or mixture of several different elastomers. Elastomeric materials are generally known in the art but illustrative examples include styrene-butadiene rubber (SBR); synthetic gums; polyisobutylene and isobutylene-isoprene copolymers; natural gums; chicle; natural rubber; jelutong; balata; guttapercha; lechi caspi; sorva; and mixtures thereof. Compositions as used in accordance with the present invention preferably comprise from about 2% to about 30%, more preferably from about 5% to about 25%, by weight, of elastomer. These levels are determined by the desired final texture of the chewing gum since when the total level of elastomer is below about 2% the base composition lacks elasticity, chewing texture, and cohesiveness whereas at levels above about 30% the formulation is hard, rubbery and maintains a tight chew. Elastomer solvents are also preferably present in compositions as used in accordance with the present invention since they aid softening of the elastomer component. Preferred examples of elastomer solvents for use herein include the pentaerythritol ester of partially hydrogenated wood rosin, pentaerythritol ester of wood rosin, glycerol ester of partially dimerized rosin, glycerol ester of polymerised rosin, glycerol ester of tall oil, wood or gum rosin, glycerol ester of partially hydrogenated rosin, methyl ester of partially hydrogenated rosin, and mixtures thereof. Compositions as used in accordance with the present invention preferably comprise from about 2% to about 50%, more preferably from about 10% to about 35%, by weight, of elastomer solvent.

Lozenges for use in accordance with this invention can be prepared, for example, by art-recognized techniques for forming compressed tablets where the disaccharide is dispersed on a compressible solid carrier, optionally combined with any appropriate tableting aids such as a lubricant (e.g., magnesium-stearate) and is compressed into tablets. The solid carrier component for such tableting formulations can be a saliva-soluble solid, such as a cold water-soluble starch or a monosaccharide, so that the lozenge will readily dissolve in the mouth to release the contained disaccharide acid in saliva solution for contact with and absorption by the oral/pharyngeal mucosa when the lozenge is held in the mouth. The pH of the above-described formulations can range from about 4 to about 8.5.

Lozenges for use in accordance with the present invention can also be prepared utilizing other art-recognized solid unitary dosage formulation techniques.

A mouth wash or mouth rinse as used in accordance with the present invention could preferably be as follows:

| | | |
|---|---|---|
| A | Olium menthae | 1.2 parts |
| | Tinctura Amicae | 3.0 parts |
| | Tinctura Myrrhae | 3.0 parts |
| | Tween | 5.0 parts |
| B | Spiritus 90% | 50.0 parts |
| C | Sodium Benzoate | 0.2 parts |
| | Sweetening agent (e.g. aspartane) Agua destilata ad 100, | 0.02 parts |

A is to be well mixed, B is added under stirring and C is added subsequently. The resulting clear liquid is to be filtered within 48 hours after preparation. Another preferred mouth wash is described in Example 26.

Regardless of the dosage form, liquid or solid, in one preferred embodiment of the present invention the dosage form is held in the patient's mouth for a period of time to promote contact of the microorganism or analog or fragment of a above mentioned microorganism belonging to the group of lactic acid bacteria with the patient's oral cavity.

The terms "dental floss" and "dental tape" as used herein refer to a material to dislodge and remove decomposing food material that accumulated at interproximal and subgingival surfaces and to dislodge and remove bacteria, plaque and/or calculus that accumulated in the oral cavity. The dental floss or dental tape may further contain, in addition to the microorganisms according to the present invention as described herein above, cleaners, abrasives, tartar control ingredients, whiteners, surfactants and/or active ingredients like fluorides, antimicrobials, chemotherapeutic agents or antibiotics. Further additional agents are antiplaque agents, flavouring agents and colouring agents. The dental floss or dental tape may be in any suitable form, known to the person skilled in the art, for example, in the form of PTFE (Teflon) dental flosses as described, for instance, in US 3,664,915 US 3,953,566, US 3,962,153 US 4,096,227, US 4,187,390, US 4,256,806, US 4,385,093, US 4,478,665, US 4,776,358, US 5,033,488, US 5,209,251, US 5,220,932, US 5,518,012, US 5,718,251, US 5,765,576 or US 5,911,228, in the form of monofilament interproximal devices as described, for instance, in US 3,800,812, US 4,974,615, US 5,760,117, US 5,433,226, US 5,479,952, US 5,503,842, US 5,755,243, US 5,884,639, US 6,003,525 or US 6,027,592, or in the form of biocomponent tapes. Preferably, the dental floss or dental tape may be in the form of an elastomeric coated monofilament as described, for instance, in US 20050226820 or in the form of an oriented thermoplastic based dental tape as described, for instance, in US 20020144704.

The anticariogenic cosmetic compositions as described herein above may be used in the ambit of human oral administration as well as in the ambit of veterinary oral administration, preferably for non-human mammals, more preferably for pets. If the antcariogenic cosmetic composition is used in the ambit of veterinary oral administration, the composition may contain further ingredients suitable for such an administration, as known by a person skilled in the art.

In another aspect the present invention relates to the use of a microorganism belonging to the group of lactic acid bacteria as defined in the claims, which is able to specifically bind to a bacterium belonging to the group of mutans Streptococci as defined in the claims, for the preparation of a pharmaceutical composition for the treatment or prophylaxis of caries caused by mutans Streptococci other than Streptococcus mutans, namely S. sobrinus. Preferably, such a pharmaceutical composition comprises a microorganism or a derivative or mutant or fragment thereof as described above. More preferably, a pharmaceutical composition further comprises a pharmaceutical acceptable carrier or excipient.

Pharmaceutical compositions comprise a therapeutically effective amount the above mentioned microorganism belonging to the group of lactic acid bacteria or a derivative, mutant or fragment of said microorganism described in connection with the composition as used in accordance with the present invention and can be formulated in various forms, e.g. in solid, liquid, powder, aqueous, lyophilized form.

The pharmaceutical composition may be administered with a pharmaceutically acceptable carrier to a patient, as described herein. In a specific embodiment, the term "pharmaceutically acceptable" means approved by a regulatory agency or other generally recognized pharmacopoeia for use in animals, and more particularly in humans. A preferred pharmaceutical composition as used in accordance with the present invention does not contain lactose in a range between 1% (w/w) and 6% (w/w). It is also preferred that the pharmaceutical composition contains not more than 1%(w/w) lactose, e.g. it contains less than 1%, preferably less than 0.9% (w/w), 0.8% (w/w) lactose, etc. or that the pharmaceutical composition contains more than 6%, 7%, 8% etc. (w/w) lactose. Alternatively, but also preferred is that the pharmaceutical composition does not contain lactose.

The term "carrier" refers to a diluent, adjuvant, excipient, or vehicle with which the therapeutic is administered. Such a carrier is pharmaceutically acceptable, i.e. is non-toxic to a recipient at the dosage and concentration employed. It is preferably isotonic, hypotonic or weakly hypertonic and has a relatively low ionic strength, such as provided by a sucrose solution. Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. Suitable pharmaceutical excipients include starch, glucose, sucrose, gelatin, malt, rice, flour, chalk, silica gel, sodium stearate, glycerol monostearate, talc, sodium ion, dried skim milk, glycerol, propylene, glycol, water, ethanol and the like. The excipient may contain lactose as described herein above, most preferably it is lactose-free. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, starch, magnesium stearate, sodium saccharine, cellulose, magnesium carbonate, etc. Examples of suitable pharmaceutical carriers are described in "Remington's Pharmaceutical Sciences" by E.W. Martin. Skim milk, skim milk powder, non-milk or non-lactose containing products may also be employed. The skim milk powder is conventionally suspended in phosphate buffered saline (PBS), autoclaved or filtered to eradicate proteinaceous and living contaminants, then freeze dried heat dried, vacuum dried, or lyophilized. Some other examples of substances which can serve as pharmaceutical carriers are sugars, such as glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; calcium carbonate; vegetable oils, such as peanut oils, cotton seed oil, sesame oil, olive oil, com oil and oil of theobroma; polyols such as propylene glycol, glycerine, sorbitol, manitol, and polyethylene glycol; agar, alginic acids; pyrogen-free water; isotonic saline; cranberry, extracts and phosphate buffer solution; skim milk powder; as well as other non-toxic compatible substances used in pharmaceutical formulations such as Vitamin C, estrogen and echinacea, for example. Wetting agents and lubricants such as sodium lauryl sulfate, as well as colouring agents, flavouring agents, lubricants, excipients, tabletting agents, stabilizers, anti-oxidants and preservatives, can also be present.

Preferably, the oral formulation contains lactose as described herein and is most preferably lactose-free. Various carriers and/or excipients suitable for oral administration which are well known in the art may be used for the purpose of this invention. The non-cariogenic composition may, if desired, further contain various known additives such as, for example, preservatives, hardening agents, lubricants, emulsifiers, stabilizers, essence and the like. Such compositions will contain a therapeutically effective amount of the aforementioned compounds, preferably in purified form, together with a suitable amount of carrier so as to provide the form for proper administration to the patient. The formulation should suit the mode of administration.

Generally, the ingredients are supplied either separately or mixed together in unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline.

The pharmaceutical composition of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

In vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant. Adjuvants may be selected from the group consisting of a chloroquine, protic polar compounds, such as propylene glycol, polyethylene glycol, glycerol, EtOH, 1-methyl L-2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives. These compounds are added in conditions respecting pH limitations. The composition as used in accordance with the present invention can be administered to a vertebrate. "Vertebrate" as used herein is intended to have the same meaning as commonly understood by one of ordinary skill in the art. Particularly, "vertebrate" encompasses mammals, and more particularly humans.

The term "administered" means administration of a therapeutically effective dose of the aforementioned composition. By "therapeutically effective amount" is meant a dose that produces the effects for which it is administered, preferably this effect is anticariogenic. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt %. The agents maybe administered alone or in combination with other treatments.

The administration of the pharmaceutical composition can be done in a variety of ways as discussed above, including, but not limited to, orally, subcutaneously, intravenously, intra-arterial, intranodal, intramedullary, intrathecal, intraventricular, intranasally, intrabronchial, transdermally, intranodally, intrarectally, intraperitoneally, intramuscularly, intrapulmonary, vaginally, rectally, or intraocularly.

Preferably the administration is orally or buccal. The attending physician and clinical factors will determine the dosage regimen. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors.

The dosages are preferably given once a week, however, during progression of the treatment the dosages can be given in much longer time intervals and in need can be given in much shorter time intervals, e.g., daily. In a preferred case the immune response is monitored using herein described methods and further methods known to those skilled in the art and dosages are optimized, e.g., in time, amount and/or composition. Progress can be monitored by periodic assessment. The pharmaceutical composition of the invention may be administered locally or systemically. It is also envisaged that the pharmaceutical compositions are employed in co-therapy approaches, i.e. in co-administration with other medicaments or drugs, for example other drugs for preventing, treating or ameliorating caries, which are described herein.

In another preferred embodiment the present invention relates to the use of a microorganism belonging to the group of lactic acid bacteria as defined in the claims, which is able to specifically binding to a bacterium belonging to the group of mutans Streptococci as defined in the claims, for the preparation of an anticariogenic composition for the treatment or prophylaxis of caries caused by mutans Streptococci other than Streptococcus mutants, namely S. sobrinus, wherein the anticariogenic composition is a foodstuff or feedstuff. Preferably an anticariogenic composition in the form of a foodstuff or feedstuff is a food or feed composition comprising a microorganims, mutant, derivative or fragment thereof as described herein above further comprising an orally acceptable carrier or excipient. More preferably, the microorganism, mutant, derivative or fragment thereof is a deposited microorganism as described herein above, or a mutant, derivative or fragment thereof.

"Food" or "feed" comprises any latable, palatable and/or drinkable stuff for mammals, for example, humans or animals, e.g., pets as described herein. Food and feedstuff is described herein elsewhere. An "orally acceptable carrier" is described herein above and is preferably not toxic and of food and/or feed grade. Yet, this term also encompasses the carriers mentioned in connection with the pharmaceutical composition as used in accordance with the present invention. A preferred food or feed composition as used in accordance with the present invention does not contain lactose in a range between 1% (w/w) and 6% (w/w). It is also preferred that the food or feed composition contains not more than 1% (w/w) lactose, e.g. it contains less than 1%, preferably less than 0.9% (w/w), 0.8% (w/w) lactose, etc. or that the food or feed composition contains more than 6%, 7%, 8% etc. (w/w) lactose. Alternatively, but also preferred is that the food or feed composition does not contain lactose.

In accordance with the present invention, the term "foodstuff" encompasses all eatable and drinkable food and drinks. Accordingly, the microorganism, derivative, analog or fragment thereof may be included in a food or drink. These are, for example, gum, spray, beverage, candies, infant formula, ice cream, frozen dessert, sweet salad dressing, milk preparations, cheese, quark, lactose-free yogurt, acidified milk, coffee cream or whipped cream and the like.

Milk-based products are envisaged within the framework of the invention. Milk is however understood to mean that of animal origin, such as cow, goat, sheep, buffalo, zebra, horse, donkey, or camel, and the like. The milk may be in the native state, a reconstituted milk, a skimmed milk or a milk supplemented with compounds necessary for the growth of the bacteria or for the subsequent processing of fermented milk, such as fat, proteins of a yeast extract, peptone and/or a surfactant, for example. The term milk also applies to what is commonly called vegetable milk, that is to say extracts of plant material which have been treated or otherwise, such as leguminous plants (soya bean, chick pea, lentil and the like) or oilseeds (colza, soya bean, sesame, cotton and the like), which extract contains proteins in solution or in colloidal suspension, which are coagulable by chemical action, by acid fermentation and/or by heat. Finally, the word milk also denotes mixtures of animal milks and of vegetable milks.

Where the microorganism of this invention or derivative or analog or fragment thereof are added to yogurt and the like having similar contents, it is sufficient to add the microorganism of this invention at a concentration of about 10⁵ -10⁷ cells/ml. In such a case, it is possible to completely prevent or inhibit dental caries induced by cariogenic strains of mutans Streptococci without significant side effect upon the quality of the drink per se.

Such food drink or feed can be produced by a general method for producing foods and drinks or feeds, including adding the active ingredient to a raw or cooked material of the food, drink or feed. The food, drink or feed in accordance with the present invention can be molded and granulated in the same manner as generally used for foods, drinks or feeds. The molding and granulating method includes granulation methods such as fluid layer granulation, agitation granulation, extrusion granulation, rolling granulation, gas stream granulation, compaction molding granulation, cracking granulation, spray granulation, and injection granulation, coating methods such as pan coating, fluid layer coating, and dry coating, puff dry, excess steam method, foam mat method, expansion methods such as microwave incubation method, and extrusion methods with extrusion granulation machines and extruders.

The food, drink or feed to be used in the present invention includes any food, drink or feed which comprises the microorganism of the invention, derivative or fragment thereof as active ingredient. The active ingredient in the food, drink or feed is not specifically limited to any concentration as long as the resulting food, drink or feed can exert its activity of specifically binding to mutans Streptococci. The concentration of the active ingredient is preferably 0.001 to 100 % by weight, more preferably 0.01 to 100 % by weight and most preferably 0.1 to 100 % by weight of the food, drink or feed comprising such active ingredient or with respect to the cell number those described herein.

Specific foods or drinks, to which the active ingredient is added, include, for example, juices, refreshing drinks, soups, teas, sour milk beverages, dairy products such as fermented milks, ices, butter, cheese, processed milk and skim milk, meat products such as ham, sausage, and hamburger, fish meat cake products, egg products such as seasoned egg rolls and egg curd, confectioneries such as cookie, jelly, snacks, and chewing gum, breads, noodles, pickles, smoked products, dried fishes and seasonings. The form of the food or drink includes, for example, powder foods, sheet-like foods, bottled foods, canned foods, retort foods, capsule foods, tablet foods and fluid foods.

The food or drink with an activity to specifically bind to mutans Streptococci to be ingested by infants, are preferably nutritious compositions for infants. Such nutritious composition for infants includes modified milk prepared for infants, protein-decomposed milk, specific nutritionally modified milk or baby foods and foods prepared for toddlers. The form of the nutritious composition for infants includes but is not specifically limited to powder milks dried and pulverized and baby foods and also include general foods such as ice cream, fermented milk, and jelly for infantile ingestion.

The nutritious composition for infants in accordance with the present invention is principally composed of protein, lipid, saccharide, vitamins and/or minerals. In the nutritious composition, the active ingredient is blended with these components.

The protein includes milk proteins such as skim milk, casein, cheese whey, whey protein concentrate and whey protein isolates and their fractions such as alpha s - casein, beta-casein, alpha-lactoalbumin and beta-lactoglobulin. Further, egg protein such as egg yolk protein, egg white protein, and ovalbumin, or soybean protein such as defatted soybean protein, separated soybean protein, and concentrated soybean protein can be used. Other than these, proteins such as wheat gluten, fish meat protein, cattle meat protein and collagen may also be used satisfactorily. Further, fractions of these proteins, peptides from the acid or enzyme treatment thereof, or free no acids maybe used satisfactorily as well. The free amino acids can serve as nitrogen sources and can additionally be used to give specific physiological actions. Such free amino acids include, for example, taurine, arginine, cysteine, cystine and glutamine. The lipid includes animal fats and oils such as milk fat, lard, beef fat and fish oil, vegetable oils such as soybean oil, rapeseed oil, com oil, coconut oil, palm oll, palm kernel oil, safflower oil, perilla oil, linseed oil, evening primrose oil, medium chain fatty acid triglyceride, and cotton seed oil, bacterially generated fats and oils, and fractionated oils thereof, hydrogenated oils thereof, and ester exchange oils thereof. The amount of lipid to be blended varies depending on the use.

The saccharide includes, for example, one or more of starch, soluble polysaccharides, dextrin, monosaccharides such as sucrose, lactose as described herein, maltose, glucose, and fructose and other oligosaccharides. The total amount of such saccharide is preferably 40 to 80 % by weight to the total solid in the nutritious composition. Further, artificial sweeteners such as aspartame may be used satisfactorily. The amount of an artificial sweetener is appropriately 0.05 to 1.0 % by weight per the total solid in the nutritious composition.

The vitamins include, but are not limited to, lycopene as an essential component and additionally include, for example, vitamins such as vitamin A, vitamin B group, vitamins C, D, and E and vitamin K group, folic acid, pantothenic acid, niootinamide, carnitine, choline, inositol and biotin as long as such vitamins can be administered to infants. Such vitamins are preferably from 10 mg to 5 g by weight per the total solid in the nutritious composition for infants.

Further, the minerals include calcium, magnesium, potassiw, sodium, iron, copper, zinc, phosphorus, chlorine, manganese, selenium and iodine. Such minerals are preferably from 1 mg to 5 g by weight per the total solid in the nutritious composition for infants.

Other than those components described above, the nutritious composition for infants as used in accordance with the present invention may be blended with any component desirably blended in nutritious compositions, for example, dietary fiber, nucleotides, nucleic acids, flavors, and colorants.

The food or drink as used in accordance with the present invention can be used as a health food or drink or a functional food or drink to prevent and/or treat caries caused by mutans Streptococci other than Streptococcus mutans.

When the food or drink according to the present invention is ingested, the amount to be ingested is not specifically limited. The amount to be ingested is generally 0.1 to 50 g, preferably 0.5 g to 20 g daily, based on the total amount of active ingredient. The food or drink is continuously ingested at this amount for a period from a single day up to 5 years, preferably from 2 weeks to one year. Herein, the amount ingested can be adjusted to an appropriate range depending on the severity of the symptom of the individual ingesting the food or drink, the age and body weight thereof, and the like.

The feed as used in accordance with the present invention maybe any feed comprising the active ingredient. The feed includes, for example, pet feeds for dogs, cats and rats, cattle feeds for cows and pigs, chicken feeds for chicken and turkeys, and fish cultivation feeds for porgy and yellowtail.

The feed can be produced by appropriately blending the active ingredient as described herein above in a raw feed material including, for example, cereals, brans, oil-seed meals, animal-derived raw feed materials, other raw feed materials and purified products.

The cereals include, for example, mile, wheat, barley, oats, rye, brown rice, buckwheat, fox-tail millet, Chinese millet, Deccan grass, com, and soybean.

The brans include, far example, rice bran, defatted rice bran, bran, lowest-grade flour, wheat germ, barley bran, screening pellet, com bran, and com germ.

The oil-seed meals include, for example, soybean meal, soybean powder, linseed meal, cottonseed meal, peanut meal, safflower meal, coconut meal, palm meal, sesame meal, sunflower meal, rapeseed meal, kapok seed meal and mustard meal. The animal-derived raw feed materials include , for example, fish powders, import meal, whole meal, and coast meal, fish soluble, meat powder, meat and bone powder, blood powder, decomposed hair, bone powder, byproducts from butchery, feather meal, silkworm pupa, skim milk, casein, dry whey and krill.

Other raw feed materials include, for example, plant stems and leaves such as alfalfa, hey cube, alfalfa leaf meal, and locust leaf powder, byproducts from com processing industries, such as corn gluten meat, corn gluten feed and corn steep liquor, starch, sugar, yeast, byproducts from fermentation industry such as beer residue, malt root, liquor residue and soy sauce residue, and agricultural byproducts such as citrus processed residue, soybean curd residue, coffee residue, and cocoa residue, cassava, horse bean, guar meal, seaweed, spirulina and chlorella.

The purified products include, for example, proteins such as casein and albumin, amino acids, starch, cellulose, saccharides such as sucrose and glucose, minerals and vitamins.

In case of providing to animals the feed according to the present invention, the amount of the feed to be ingested is not specifically limited but is preferably, for example, 0.1 mg to 50 g per I kg body weight per day, preferably 0.5 mg to 20 g per 1 kg body weight per day, based on the amount of the active ingredient. The feed is continuously ingested at this amount for a period from a single day up to 5 years, preferably from 2 weeks to one year. Again, the amount ingested can be adjusted to an appropriate range depending on the species, age and body weight of the animal ingesting the feed, and the like.

In a further embodiment, the present invention relates to the use of a microorganism belonging to the group of lactic acid bacteria namely as defined in the claims, which is able to specifically binding to a bacterium belonging to the group of mutans Streptococci as defined in the claims, for the preparation of an anticariogenic composition for the treatment or prophylaxis of caries caused by mutans Streptococci other than Streptococcus mutan, namely S. sobrinus, wherein the the anticariogenic composition is an additive for foods, drinks and feeds. Preferably, the additive for foods, drinks and feeds is, due to the presence of a microorganism or derivative or mutant or fragment thereof as described herein above is, inter alia, capable of specifically binding to mutans Streptococci so as to prevent and/or treat caries caused by mutans Streptococci other than Streptococcus mutans, specifically S. sobrinus. Preferably, the microorganism, mutant, derivative or fragment thereof is a deposited microorganism as described herein above, or a mutant, derivative or fragment thereof. The additive for foods or drinks includes the additive for nutritious compositions for infants.

The additive for foods can be produced by a general method for producing additives for foods, drinks or feeds. If necessary, additives for general use in foods, drinks or feeds, for example, additives described in Food Additive Handbook (The Japan Food Additives Association; issued on January 6, 1997) may be added satisfactorily, including sweeteners, colorants, preservatives, thickeners and stabilizers, anti-oxidants, color fixing agents, bleaches, antiseptics, gum base, bitters, enzymes, brightening agents, acidifier, seasonings, emulsfiers, enhancers, agents for manufacture, flavors, and spice extracts. Further, conventional saccharides, starch, inorganic materials, plant powders, excipients, disintegrators, lubricants, binders, surfactants, and plasticizers mentioned previously for pharmaceutical tablets may be added satisfactorily.

The additives include the following additives.

The sweeteners include aspartame, licorice, stevia, xylose and rakanka (Momordica grosvenon fruit). The colorants include carotenoid and turmeric oleoresin, flavonold, caramel color, spirulina color, chlorophyll, purple sweet potato color, purple yam color, perilla color, and blueberry color.

The preservatives include, for example, sodium sulfite, benzoates, benzoin extract, sorbates, and propionates. The thickeners and stabilizers include, for example, gums such as gum arable and xanthan gum, alginates, chitin, chitosan, aloe extract, guar gum, hydroxypropyl cellulose, sodium casein, corn starch, carboxymethyl cellulose, gelatin, agar, dextrin, methyl cellulose, polyvinyl alcohol, microfiber cellulose, microcrystalline cellulose, seaweed cellulose, sodium polyacrylate, sodium polyphosphate, carrageenan or yeast cell wall.

The anti-oxidants include, for example, vitamin C group, sodium ethylenediaminetetraacetate, calcium ethylenediaminetetraacetate, erythorbic acid, oryzanol, catechin, quercetin, clove extract, enzyme-treated rutin, apple extract, sesame seed extract, dibutylhydroxytoluene, fennel extract, horseradish extract, water celery extract, tea extract, tocopherols, rapeseed extract, coffee bean extract, sunflower seed extract, ferulio acid, butylhydroxyanisole, blueberry leaf extract. propolis extract, pepper extract, garden balsam extract, gallic acid, eucalyptus extract, and rosemary extract.

The color fixing agents include, for example, sodium nitrite. The bleaches include, for example, sodium sulfite.

The antiseptics include, for example, o-phenyl phenol. The gum base includes, for example, acetylricinoleate methyl, urushi wax, ester gum, elemi resin, urucury wax, kaurigum, carnaubawax, glycerin fatty acid ester, spermaceti wax, copaibabalsam, copal resin, rubber, rice bran wax, cane wax, shellac, jelutong, sucrose fatty acid ester, depolymerized natural rubber, paraffin wax, fir balsam, propylene glycol fatty acid ester, powdered pulp, powdered rice hulls, jojoba oil, polyisobutylene, polybutene, microcrystalline wax, mastic gum, bees wax and calcium phosphate. The bitters include, for example, iso-alpha-bitter acid, caffeine, kawaratake (Coriolus versieolor) extract, redbark cinchona extract, Phelladendron bark extract, gentian root extract, spice extracts, enzymatically modified naringin, Jamaica cassia extract, theabromine, naringin, cassia extract, absinth extract, isodonis extract, olive tea, bitter orange (Citrus aurantium) extract, hop extract and wormwood extract.

The enzymes include, for example, amylase, trypsin or rennet.

The brightening agents include, for example, urushi wax and japan wax. The acidifier include, for example, adipic acid, itacania acid, citric acids, succinic acids, sodium acetate, tartaric acids, carbon dioxide, lactic acid, phytic acid, fumario acid, malic acid and phosphoric acid. The seasonings include, for example, amino acids such as asparagine, aspartic acid, glutamic acid, glutamine, alanine, isoleucine, glycine, serine, cystine, tyrosine, leucine, and praline, nucleic acids such as sodium inosinate, sodium uridinate, sodium guanylate, sodium cytidylate, calcium ribonucleotide and sodium ribonucleotide, organic acids such as citric acid and succinic acid, potassium chloride, sodium chloride-decreased brine, crude potassium chloride, whey salt, tripotassium phosphate, dipotassium hydrogen phosphate, potassium dihydrogen phosphate, disodium hydrogen phosphate, sodium dihydrogen phosphate, trisodium phosphate and chlorella extract.

The enhancers include, for example, zinc salts, vitamin C group, various amino acids, 5-adenylic acid, iron chloride, hesperidin, various calcined calcium, various non-calcined calcium, dibenzoylthiamine, calcium hydroxide, calcium carbonate, thiamine' hydrochloride salt, Dunallella. Oarotene, tocopherol, nicotinic acid, carrot carotene, palm oil carotene, calcium pantothenate, vitamin A, hydroxyproline, calcium dihydrogen pyrophosphate, ferrous pyrophosphate, ferric pyrophosphate, ferritin, heme iron, menaquinone, folic acid and riboflavine.

The agents for manufacture include, for example, processing auxiliaries such as acetone and ion exchange resin. The flavors include, for example, vanilla essence and the spice extracts include, for example, capsicum extract.

These various additives can be added to the active ingredient, taking into consideration the mode of administration, in accordance with the present invention. The anticariogenic composition as used in accordance with the present invention encompasses an amount of the above mentioned microorganism belonging to the group of lactic acid bacteria or a derivative or mutant thereof or analog or fragment thereof. Preferably, the microorganism, mutant, derivative or fragment thereof is a deposited microorganism as described herein above, or a mutant, derivative or fragment thereof. It is envisaged that the compositions and in particular the anticariogenic composition comprise the above mentioned microorganism belonging to the group of lactic acid bacteria in the form of a probiotic microorganism. Namely, in addition to the probiotic effect, the above mentioned probiotic microorganism belonging to the group of lactic acid bacteria is useful for treating and/or preventing caries caused by mutans Streptococci other than Streptococcus mutans. The amount of said probiotic microorganism is high enough to significantly positively modify the condition to be treated, preferably caries, but low enough to avoid serious side effects (at a reasonable benefit/risk ratio), within the scope of sound medical judgment. An effective amount of said probiotic microorganism will vary with the particular goal to be achieved, the age and physical condition of the patient being treated, the severity of the underlying disease, the duration of treatment, the nature of concurrent therapy and the specific microorganism employed. The effective amount of said probiotic microorganism will thus be the minimum amount which will provide the desired specific binding to mutans Streptococci. The presence of, for example, 1 x 10⁹ bacteria, as viable or non-viable whole cells, in 0.05 ml solution of phosphate buffered saline solution, or in 0.05 ml of suspension of agar, or the dry weight equivalent of cell wall fragments, is effective when administered in quantities of from about 0.05 ml to about 20 ml.

A decided practical advantage is that the probiotic organism may be administered in a convenient manner such as by the oral route. Depending on the route of administration, the active ingredients which comprise said probiotic organisms may be required to be coated in a material to protect said organisms from the action of enzymes, acids and other natural conditions which may inactivate said organisms. In order to administer probiotic organisms by other than parenteral administration, they should be coated by, or administered with, a material to prevent inactivation. For example, probiotic organisms may be co-administered with enzyme inhibitors or in liposomes. Enzyme inhibitors include pancreatic trypsin inhibitor, diisopropylfluorophosphate (DFP) and trasylol. Liposomes include water-in-oil-in-water P40 emulsions as well as conventional and specfically designed liposomes which transport lactobacilli or their by-products to the urogenital surface. Dispersions can also be prepared, for example, in glycerol, liquid polyethylene glycols, and mixtures thereof, and in oils. Generally, dispersions are prepared by incorporating the various sterilized probiotic organisms into a sterile vehicle which contains the basic dispersion medium and the required other ingredients from those enumerated above. In the case of sterile powders for the preparation of sterile injectable solutions, the preferred methods of preparation are vacuum-drying and the freeze-drying technique which yield a powder of the active ingredient plus any additional desired ingredient from previously sterile-filtered solution thereof. Additional preferred methods of preparation include but are not limited to lyophilization and heat-drying.

The anticariogenic composition also encompasses products intended to be administered orally, or buccal, which comprise an acceptable pharmaceutical carrier as described herein to which, or onto which, cells of the above mentioned microorganism belonging to the group of lactic acid bacteria is added in fresh, concentrate or dried form, for example. Of course, also a derivative or analog or fragment of said microorganism can be added or any combination of said microorganism, derivative and/or fragment thereof which are disclosed herein. These products may be provided in the form of an ingestible suspension, a gel, a diffuser, a capsule, a hard gelatin capsule, a syrup, or in any other galenic form known to persons skilled in the art.

When the probiotic organisms are suitably protected as described above, the active compound may be orally administered, for example, with an inert diluent or with an assimilable edible carrier, or it may be enclosed in hard or soft shell gelatin capsule, or it may be compressed into tablets designed to pass through the stomach (i.e., enteric coated), or it may be incorporated directly with the food of the diet. For oral therapeutic administration, the probiotic organisms may be incorporated with excipients and used in the form of ingestible tablets, buccal tablets, troches, capsules, elixirs, suspensions, syrups, wafers, and the like. Compositions or preparations according to the present invention are prepared so that an oral dosage unit form contains, for example, about 1 x 10⁹ viable or non-viable e.g., lactobacilli per ml. The probiotic organism is compounded for convenient and effective administration in effective amounts with a suitable pharmaceutically or food acceptable carrier in dosage unit form as hereinbefore disclosed. A unit dosage form can, for example, contain the principal active compound in an amount approximating 10⁹ viable or non-viable, e.g., lactobacilli, per ml. In the case of compositions containing supplementary ingredients such as prebiotics, the dosages are determined by reference to the usual dose and manner of administration of the said ingredients.

Another aspect in connection with the present invention relates to a method of prophylaxis or treatment of caries caused by mutans Streptococci other than Streptococcus mutans. Preferably the method of prophylaxis or treatment comprises administering to a subject a microorganism belonging to the group of lactic acid bacteria, characterized in that said microorganism is capable of specifically binding to a bacterium belonging to the group of mutans Streptococci or a mutant, derivative or fragment of said microorganism as described herein above. More preferably, the microorganism is a microorganism as described herein above, even more preferably, the microorganims is a lactobacillus deposited at the DSMZ, as described herein above.

The derivative or fragment of the above mentioned microorganism belonging to the group of lactic acid bacteria may be any derivative or fragment as described herein above.

Preferably, the subject to be treated is an animal. More preferably, the animal is a mammal, even more preferably the mammal is a pet mammal. In a preferred embodiment, the pet is a dog, a cat, a hamster, a monkey, a rat or a mouse. In another preferred embodiment the animal is a cattle, a horse, a swine, a donkey, a sheep or a goat. In another preferred embodiment the mammal is a human being.

Preferably, a mutans Streptococcus is a microorganism belonging to the species Streptococcus mutans, Streptococcus sobrinus, Streptococcus cricetus, Streptococcus ratti, Streptococcus ferus or Streptococcus macacae. Even more preferably, a mutans Streptococcus relates to a microorganism belonging to Streptococcus mutans serotype c (DSMZ 20523) Streptococcus mutans serotype e (NCTC 10923) Streptococcus mutans serotype f (NCTC 11060), Streptococcus sobrinus DSM 20742 Streptococcus ratti DSM 20564, Streptococcus cricetus DSM 20562, Streptococcus ferus DSM 20646 or Streptococcus macacae DSM 20714. The caries to be treated or prevented is caused by mutans Streptococci other than Streptococcus mutans, preferably, the caries is caused by at least one bacterium selected from the group consisting of, Streptococcus sobrinus, Streptococcus cricatus, Streptococcus ratti, Streptococcus ferus and Streptococcus macacae. The administration of a microorganism belonging to the group of lactic acid bacteria as described above in the context of the method of treatment or prophylaxis in connection with the present invention may be carried out in any suitable form known to the person skilled in the art. Preferably, the administration encompasses the use and application of compositions as described herein above, which may optionally contain, for example, pharmaceutical or cosmetic carriers or excipients as described herein above. The dosage and time course of the administration may be established according any suitable information known to the person skilled in the art. Preferably, said dosage and time course may be established as described herein above.

The invention is illustrated by Figures 1 to 3 as described in the following:
**Figure 1** shows the aggregation of Streptococcus mutans by Lactobacillus species. In particular, the figure depicts a mixture of an aggregating Lactobacillus with S. mutans (left tube) in comparison with a mixture of a non-aggregating Lactobacillus with S. mutans (right tube). The experiment has been performed as described in Example 4 and the tubes were left undisturbed for 20 minutes to allow the aggregates to settle.

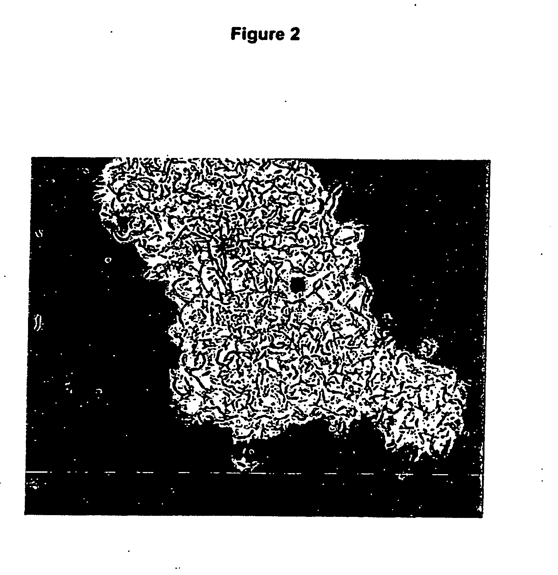

**Figure 2** shows a microscopic picture of the aggregate between Lactobacillus and S. mutans shown in Figure 1 (left tube). The picture was taken at a 1000-fold magnification using a phase-contrast microscope.

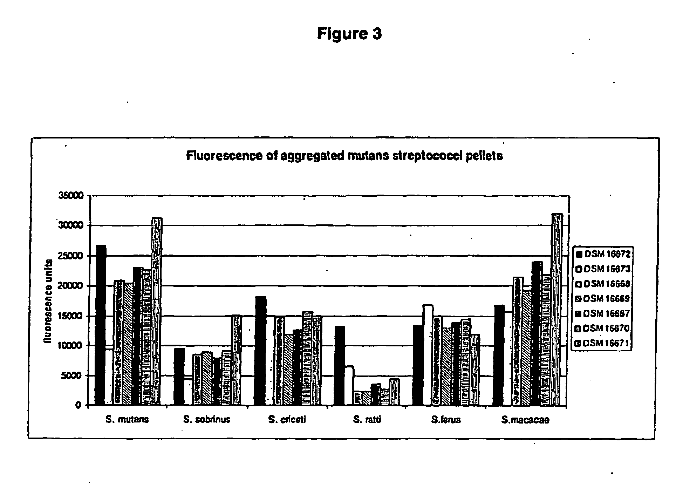

**Figure 3** shows the aggregation of different mutans Streptococci by lactobacilli. The assay was carried out as described in Example 5. Briefly, the mutans Streptococci were stained using a fluorescence stain. After aggregation of the Streptococci by the lactobacilli, the resulting pellets were separated by centrifugation. The amount of fluorescence of the pellets was taken as a measure for the amount of aggregated mutans Streptococci.

A better understanding of the present invention and of its many advantages will be had from the following examples, offered for illustrative purposes only.

### Example 1

### Storage and growth

Storage and growth of strains can occur according to ordinary procedures. For example, strains can be stored as frozen stocks at -80°C. 1 ml of a culture can be grown to stationary phase (OD600/mL 4 - 8) in MRS-Medium and mixed with 500 µl of a sterile 50% glycerine solution and frozen. Cultures of mutans Streptococci can be grown in TSY-media to stationary phase (OD600/mL 1-2) and be treated as mentioned above.

Cultivation of mutans Streptococci (S. mutans, S. sobrinus, S. ratti, S. cricetus, S. ferus or S. macacae) as well as cultivation of ladobacilli can be done in 5 ml in closed Falcon tubes at 37°C without shacking over night.

In particular, the strains used in the present application were stored as frozen stocks at -80°C. 1 ml of a culture grown to stationary phase (OD600/mL 4 - 8) in MRS-broth was mixed with 500 µl of a sterile 50% glycerol solution and frozen.

In particular, cultures of mutans Streptococci were grown in TSY-broth to stationary phase (OD600/mL 1-2) and treated as mentioned above.

Cultivation of mutans Streptococci (S. mutans (DSM 20523, serotype c; NCTC 10923, serotype e; NCTC 11060, serotype f), S. sobrinus DSM 20742, S. ratti DSM 20564, S. cricetus DSM 20562, S. ferus DSM 20646 or S. macacae DSM 20714) and cultivation of lactobacilli was done in 5 ml in closed Falcon tubes at 37°C without shacking over night. For the fluorescence assays as described in Example 5 S. mutans DSM 20523 was used.

For an aggregation assay the lactobacilli were grown in MRS-medium. 5 ml MRS-medium were inoculated with 10µL of the stock and incubated for 3 days at 37°C under aerobic conditions. The optical density of the culture at 600 nm (OD₆₀₀) was measured. The culture was then diluted to an OD₆₀₀ of 2 using PBS-buffer. The mutans Streptococci were grown in 7 ml TSY-medium. 7 ml of TSY-medium were inoculated with 10 µl of the stock and incubated at 37°C under anaerobic conditions.

### Example 2

### Taxonomic classification of strains

The taxonomic classification of the strains was done according to their carbohydrate fermentation pattern. This was determined using the API 50 CH (bioMerieux, France) system and analyzed using APILAB PLUS software version 3.3.3 (bioMerieux, France).

### Example 3

### Staining of cells

After the lactobacilli and the mutans Streptococci were grown as described in Example 1, the mutans Streptococci were stained using a fluorescence strain. For this, the OD₆₀₀ of the culture was measured. The culture was harvested by centrifugation at 3200 x g for 5 min. The pellet was resuspended in PBS-buffer. The amount of buffer was calculated so that the resulting suspension had an OD600 of 4.2 ml of that suspension wee mixed with 2µl of a CFDA-SE solution (Invitrogen, USA) that was prepared according to the manufacturers instructions. Staining of the cells was carried out by incubating the mixture for 2 h at 37°C. The stained cells were harvested by centrifugation at 3200 x g for 5 min. The cells were subsequently reuspended in 2 ml PBS-buffer.

### Example 4

### Pelleting aggregation assay of mutans Streptococci

For the assay, mixing of lactobacilli and mutans Streptococci was done in volumetric ratios of 3:1 to 60:1 (mutans Streptococci : lactobacilli), this corresponds to a ratio of colony forming units from 1:50 to 1:2,5.

An optical density measured at a wavelength of 600 nm in 1 ml means preferably for mutans Streptococci 3 x 10⁸ colony forming units and for ladobacilli preferably 7 x 10⁹ colony forming units. Mixing was done in 2 mL volume in 15 mL Falcon tubes. The culture suspensions were diluted with PBS-buffer to obtain the volumetric ratios mentioned above while keeping the final volume at 2 ml. The mixture was vortexed for 15 seconds. An aggregation is visible as an immediate turbidity of the suspension. The tubes were left undisturbed for 20 min, after that period of time the aggregates settle as a visible pellet whereas non-aggregating mixtures stay in suspension.

The formed aggregates were separated by centrifugation at 500 x g for 30 seconds. Afterwards, the amount of aggregation was quantified by measuring the amount of non-aggregated cells that were left in the supernatant. Correspondingly, 1 ml of the supernatant was carefully removed to measure the optical density. The optical density was measured at 600 nm. The value after subtraction of the respective control experiment without lactobacilli represents the amount of cells that have not been aggregated.

As a control, self-aggregation of the respective Lactobacillus strain and the mutans Streptococcus strains was always investigated by performing the test with only the Lactobacillus or the mutans Streptococcus strain added to the tube. An aggregation of S. mutans by Lactobacillus is shown in Figures 1 (left tube) and 2.

The lactobacilli strains as described herein above, in particular those deposited with the DSMZ, exhibited aggregation of all S. mutans serotypes without showing a self-aggregation behaviour.

### Media:

| | |
|---|---|
| **MRS-broth:** | |
| MRS-mixture (Difco, USA) | 55 g/L |
| pH: | 6.5 |
| TSY-broth: | |
| TSY-mixture (Difco,USA) | 30 g/L |
| Yeast extract (Deutsche Hefewerke, Germany) | 3 g/L |

### Buffer:

| PBS-buffer: | |
|---|---|
| Na₂HPO₄*2H₂0 | 1.5 g/L |
| KH₂PO₄ | 0.2 g/L |
| NaCl | 8.8 g/L |

pH adjusted with HCl

### Example 5

### Fluorescence aggregation assay of mutans Streptococci

For the assay, suspension of the respective lactobacillus and the respective stained mutans Streptococcus (S. mutans DSM 20523 with Lb-OB-K1 (DSM 16667), S. mutans DSM 20523 with Lb-OB-K2 (DSM 16668), S. mutans DSM 20523 with Lb-OB-K3 (DSM 16669), S. mutans DSM 20523 with Lb-OB-K4 (DSM 16670), S. mutans DSM 20523with Lb-OB-K5 (DSM 16671), S. mutans DSM 20523 with Lb-OB-K6 (DSM 16672), S. mutans DSM 20523 with Lb-OB-K7 (DSM 16673);
S. sobrinus DSM 20742 with Lb-OB-K1 (DSM 16667), S. sobrinus DSM 20742 with Lb-OB-K2 (DSM 16668), S. sobrinus DSM 20742 with Lb-OB-K3 (DSM 16669), S. sobrinus DSM 20742 with Lb-OB-K4 (DSM 16670), S. sobrinus DSM 20742 with Lb-OB-K5 (DSM 16671), S. sobrinus DSM 20742 with Lb-OB-K6 (DSM 16672), S. sobrinus DSM 20742 with Lb-OB-K7 (DSM 16673);
S. cricetus DSM 20562 with Lb-OB-K1 (DSM 16667), S. cricetus DSM 20562 with Lb-OB-K2 (DSM 16668), S. cricetus DSM 20562 with Lb-OB-K3 (DSM 16669), S. cricetus, DSM 20562 with Lb-OB-K4 (DSM 16670), S. cricetus DSM 20562 with Lb-OB-K5 (DSM 16671), S. cricetus DSM 20562 with Lb-OB-K6 (DSM 16672), S. cricetus DSM 20562 with Lb-OB-K7 (DSM 16673);
S. ratti DSM 20564 with Lb-OB-K1 (DSM 16667), S. ratti DSM 20564 with Lb-OB-K2 (DSM 16668), S. ratti DSM 20564 with Lb-OB-K3 (DSM 16669), S. ratti DSM 20564 with Lb-OB-K4 (DSM 16670), S. ratti DSM 20564 with Lb-OB-K5 (DSM 16671), S. ratti DSM 20564 with Lb-OB-K6 (DSM 16672), S. ratti DSM 20564 with Lb-OB-K7 (DSM 16673);
S. ferus DSM 20646 with Lb-OB-K1 (DSM 16667), S. ferus DSM 20646 with Lb-OB- K2 K2 (DSM 16668), S. ferus DSM 20646 with Lb-OB-K3 (DSM 16669), S. ferus DSM 20646 with Lb-OB-K4 (DSM 16670), S. ferus DSM 20646 with Lb-OB-K5 (DSM 16671), S. ferus DSM 20646 with Lb-OB-K6 (DSM 16672), S. ferus DSM 20646 with Lb-OB-K7 (DSM 16673);
S. macacae DSM 20724 with Lb-OB-K1 (DSM 16667), S. macacae DSM 20724 with Lb-OB-K2 (DSM 16668), S. macacae DSM 20724 with Lb-OB-K3 (DSM 16669), S. macacae DSM 20724 with Lb-OB-K4 (DSM 16670), S. macacae DSM 20724 with Lb-OB-K5 (DSM 16671), S. macacae DSM 20724 with Lb-OB-K6 (DSM 16672) and S. macacae DSM 20724 with Lb-OB-K7 (DSM 16673)) were mixed. 50µl of the lactobacillus suspension were added to 50µl of stained mutans Streptococci in a 96 well microtiter plate. The plate was vortexed at full speed for 12 minutes. Afterwards the plate was centrifuged at 500 x g for 10 seconds. The supernatant was carefully removed and discarded. The pellet was resuspended in 100µl of PBS-buffer

The fluorescence of the suspension was measured in a microtiterplate fluorescence reader at a wavelength of 495 nm for excitation and 525 for emission.

As controls lactobacilli alone as well as stained mutans Streptococci were treated and measured as described. The background fluorescence measured for the respective mutans Streptococci alone was subtracted from the value measured for the aggregation with the respective lactobacillus. All measurements were done in triplicate. The mutans Streptococci were aggregated by all tested lactobacilli (see Figure 3).

### Media:

| MRS-broth: | |
|---|---|
| MRS-mixture (Difco, USA) | 55 g/L |
| pH: | 6.5 |

| TSY-broth: | |
|---|---|
| TSY-mixture (Difco,USA) | 30 g/L |
| Yeast extract (Deutsche Hefewerke, Germany) | 3 g/L |

### Buffer:

| PBS-buffer: | |
|---|---|
| Na₂HPO₄*2H₂O | 1.5 g/L |
| KH₂PO₄ | 0.2 g/L |
| NaCl | 8.8 g/L |

pH adjusted with HCl

### Example 6

### Specificity of the aggregation towards typical members of the oral flora

The Lactobacillus cultures were grown as described in Example 1.

The oral bacteria - namely: Streptococcus salivarius subsp. thermophilus (isolated by OrganoBalance, identified by API 50 CH (Biomerieux, France) according to manufacturers instructions); Streptococcus oralis (DSMZ 20066); Streptococcus oralis (DSMZ 20395); Streptococcus oralis (DSMZ 20627); Staphylococcus epidermidis (DSMZ 1798); Staphylococcus epidermidis (DSMZ 20044); Streptococcus mitis (DSMZ 12643); Streptococcus sanguinis (DSMZ 20567) - were grown in 5 mL BHI-medium in closed 15 mL Falcon tubes at 37°C over night. Each of the above mentioned oral bacteria was preferably mixed in a volumetric ratio of 3:1 with Lactobacillus cultures and aggregation was assayed as in Example 4. For each testing of aggregation/non-aggregation only one of the aforementioned bacteria is preferably used to immediately determine the outcome of the testing.

As a control, a self-aggregation of the respective oral bacteria as well as the tested Lactobacillus strains was always investigated by performing the test with only the lactobacilli or the oral flora strains added to the tube.

The L. paracasei ssp. paracasei strains Lb-OB-K1 (DSM 16667), Lb-OB-K2 (DSM 16668), Lb-OB-K3 (DSM 16669), Lb-OB-K4 (DSM 16670), Lb-OB-K5 (DSM 16671), did not aggregate the oral bacteria mentioned above. The L. rhamnosus strains Lb-OB-K6 (DSM 16672) and Lb-OB-K7 (DSM 16673) aggregated Streptococcus salivarius subsp thermophilus.

### BHI-broth:

| | |
|---|---|
| BHI-mixture (Difco, USA) | 37 g/L |
| pH: | 7.2 |

### Example 7

### Temperature resistance of the aggregating capacity of the lactobacilli

The bacteria were grown as in Example 1.
The grown lactobacilli cultures were incubated at 121°C at 2 bar in satured steam for 20 min (autoclaved). After cooling of the autoclaved cultures to room temperature, the lactobacilli were mixed in a volumetric ratio of 1 : 3 with grown S. mutans cultures and aggregation was assayed as in Example 4 including the control experiments.
Aggregation was also assayed using the oral bacteria as outlined in Example 6.
It was found that the aggregation behaviour of the lactobacilli was not changed by the autoclaving procedure towards the tested S. mutans serotypes or towards the oral bacteria.

### Example 8

### Aggregation by heat-inactivated lactobacilli

The lactobacilli were grown as described in Example 1. Mutans Streptococci were grown and stained as described in Examples 1 and 3. The grown lactobacilli cultures were adjusted to an OD₆₀₀ of 2 as described in Example 1.1 ml of that suspension was incubated at 121°C at 2 bar for 20 min (autoclaved). After cooling of the autoclaved cultures to room temperature, aggregation was measured as described in Example 5 including control experiments.
The heat-inactivated lactobacilli still aggregated all mutans Streptococci.

### Example 9

### Dependency of the aggregation on pH-value

The bacteria were grown as in Example 1. 0.5 ml of the lactobacilli and 1.5 ml of S. mutans were harvested by centrifugation at 3200 * g for 10 min and the supernatant was discarded. The cells were resuspended in their original volume (0.5 ml and 1.5 ml, respectively) in different PBS-buffers adjusted to different pH-values. The pH-values of the buffers were adjusted to values from 7.0 to 3.0 in steps of 0.5 pH-units. Culture were resuspended in buffers of the respective pH-value that was to be used for the aggregation behaviour assay.
Afterwards the lactobacilli were preferably mixed in a volumetric ratio of 1 : 3 with S. mutans cultures and aggregation was assayed as in Example 4 including the control experiments. No visible aggregation of S. mutans by the lactobacilli occurred at pH values lower than 4.5.

### Example 10

### Dependency of the aggregation on pH-value

The lactobacilli were grown as described in Example 1. Mutans Streptococci were grown and stained as described in Examples 1 and 3. Afterwards the aggregation was assayed in different pH-values. For this purpose lactobacilli as well as streptococci were resuspended in acetate buffer adjusted to the respective pH. pH values tested were 4.0, 4.5 and 5.0. The aggregation was assayed as described in Example 5. No aggregation of mutans Streptococci occurred at pH values lower than 4.5.

### Example 11

### Sensitivity of the aggregation behaviour to lyophilisation

The bacteria were grown as in Example 1.
Aliquots of 1 ml of the lactobacilli cultures were harvested by centrifugation at 3200 * g for 10 minutes. The supernatant was discarded and the pellets were lyophilised at room temperature under vacuum for two hours. Resulting dry pellets of each tested Lactobacillus strain were stored at room temperature and at 4°C, respectively, for 1 day, 1 week, 2 weeks, 3 weeks and 4 weeks. After the storage time, lyophilised pellets were resuspended in 1 ml PBS-buffer, pH 7.0. The resuspended lactobacilli were mixed in a volumetric ratio of 1 : 3 with freshly grown S. mutans cultures and aggregation was assayed as in Example 4 including the control experiments.
The aggregation behaviour of the mentioned lactobacilli towards S. mutans was not changed by the lyophilization or the storage procedures

### Example 12

### Sensitivity of the aggregation behaviour to lyophilisation

The lactobacilli were grown as described in Example 1. Mutans Streptococci were grown and stained as described in Examples 1 and 3. The grown lactobacilli cultures were adjusted to an OD₆₀₀ of 2 as described in Example 1.1 ml of that suspension was lyophilized at room temperature under vacuum for two hours. Afterwards, the lyophilised pellets were resuspended in 1ml PBS-buffer. Aggregation was measured as described in Example 5, including control experiments.
The aggregation behaviour of the mentioned lactobacilli towards mutans Streptococci was not changed by the lyophilization.

### Example 13

### Test on protease resistance

The bacteria were grown as in Example 1.

Proteases used were Pronase E, Proteinase K, Trypsin, Chymotrypsin (all obtained from Sigma, Germany). Aliquots of 1 ml of the lactobacilli were washed in PBS-buffer by harvesting the cells by centrifugation at 3200 * g for 10 minutes and resuspending the pellet in 1 ml PBS-buffer (pH 7.0). Afterwards the cells were harvested again as described above and resuspended in PBS-buffer (pH 7.0) containing the respective protease at a final concentration of 2.5 mg/mL. The suspension was incubated for 1 hour at 37°C. Afterwards the cells were washed and resuspended in PBS-buffer (pH 7.0) as described above.
The aggregation was assayed as in Example 3 including the control experiments.
The aggregation behaviour of the mentioned lactobacilli towards S. mutans was not changed by treatment with any of the mentioned proteases.

### Example 14

### Protease susceptibility of aggregation behaviour of the lactobacilli

The lactobacilli were grown as described in Example 1. Mutans Streptococci were grown and stained as described in Examples 1 and 3. Used proteases were Pronase E, Proteinase K, Trypsin, Chymotrypsin (all obtained from Sigma, Germany). Aliquots of 1 ml of the lactobacilli were washed in PBS-buffer by harvesting the cells by centrifugation at 3200 x g for 10 min and resuspending the pellet in 1 ml PBS-buffer (pH 7.0). Afterwards, the cells were harvested again as described above and resuspended in PBS-buffer (pH 7.0) containing the respective protease at a final concentration of 2.5 mgl/ml. The suspension was incubated for 1 hour at 37°C. Afterwards, the cells were washed and resuspended in PBS-buffer (pH 7.0) as described above. The aggregation was assayed as described in Example 5 including control experiments. The aggregation behaviour of the lactobacilli towards mutans Streptococci was not changed by the treatment with any of the mentioned proteases.

### Example 15

### Ion dependency of the aggregation behaviour

The bacteria were grown as in Example 1.
Aliquots of 1 ml of the lactobacilli were washed in 1 ml 200 mM EDTA solution twice as described above. Afterwards the cells were harvested and resuspended in 1 ml PBS-buffer (pH 7.0).
The aggregation was assayed as in Example 4 and a complete loss of the aggregation ability was observed. Resuspension of the lactobacilli in 1 ml of a 2 mM calcium solution after the two times washing in 200 mM EDTA-solution restored the ability to aggregate S. mutans. Resuspension of the EDTA washed cells in up to 100 mM magnesium solution did not restore the ability to aggregate S. mutans.

### Example 16

### Ion dependency of the aggregation behaviour

The lactobacilli were grown as described in Example 1. Mutans Streptococci were grown and stained as described in Examples 1 and 3. Aliquots of 1 ml of the lactobacilli were washed in 1 ml 200 mM EDTA solution twice as described above. Afterwards the cells were harvested and resuspended in 1 ml PBS-buffer (pH 7.0).

The aggregation was assayed as described in Example 5 and a complete loss of the aggregation ability was observed. Resuspension of the lactobacilli in 1 ml of a 2 mM calcium solution after the two times washing in 200 mM EDTA-solution restored the ability to aggregate S. mutans. Resuspension of the EDTA washed cells in up to 100 mM magnesium solution did not restore the ability to aggregate mutans Streptococci.

### Example 17

### Test of aggregation In the presence of saliva

The bacteria were grown as in Example 1. 2 ml aliquots of S. mutans cultures were harvested as described above and resuspended in 2 ml of saliva. The saliva was provided by two volunteers and used immediately after winning.
The aggregation was assayed as in Example 4.
The aggregation behaviour of the mentioned lactobacilli towards S. mutans did not change in the presence of saliva.

### Example 18

### Aggregation of mutans Streptococci in the presence of saliva

Fresh saliva was sampled from volunteers. Saliva-flow was induced by chewing of sugar-free chewing gum. Volunteers collected 15 ml saliva with each sampling. The freshly collected saliva was diluted 1:2 with PBS-buffer for the assay procedure. Lactobacilli and mutans Streptococci were cultivated as described in Example 1. Mutans Streptococci were stained as described in Example 3, except that after the staining procedure the stained cells were resuspended in saliva, instead of PBS-buffer. The aggregation was measured as described in Example 5 including control experiments. The presence of saliva did not inhibit the aggregation.

### Example 19

### Lozenge composition (I)

The lozenge composition is preferably prepared as is described in Example 4 on page 8 of DE-C2 36 45 147, wherein, in addition to the ingredients mentioned in said Example 4, the above mentioned microorganism belonging to the group of lactic acid bacteria is added in an amount of 10² to 10¹², preferably 10³ to 10⁸ cells per mg of the lozenge.

### Example 20

### Lozenge composition (II)

The lozenge composition is preferably prepared as is described in Example 5 on page 8 of DE-C2 36 45 147, wherein, in addition to the ingredients mentioned in said Example 5, the above mentioned microorganism belonging to the group of lactic acid bacteria is added in an amount of 10² to 10^{12,} preferably 10³ to 10⁸ cells per mg of the lozenge.

### Example 21

### Dentifrice composition

The dentifrice composition is preferably prepared as is described in Example 3 on page 8 of DE-C2 36 45 147, wherein, in addition to the ingredients mentioned in said Example 3, the above mentioned microorganism belonging to the group of lactic acid bacteria is added in an amount of 10² to 10^{12,} preferably 10³ to 108 cells per mg of the dentifrice.

### Example 22

### Chalk-based dentifrice composition

The chalk-based dentifrice composition is preferably prepared as is described in chapter 7.1.4.4 "Rezepturbeispiel" on page 205 of the textbook "Kosmetik", W. Umbach (editor), 2nd edition, Thierne Verlag, 1995, wherein, in addition to the ingredients mentioned in said chapter on page 205, the above mentioned microorganism belonging to the group of lactic acid bacteria is added in an amount of 102 to 10¹², preferably 10³ to 10⁸ cells per mg of the chalk-based dentifrice.

### Example 23

### Gel-Dentifrice on basis of silicic acid/sodium fluoride

The gel-dentifrice on basis of silicic acid/sodium fluoride dentifrice composition is preferably prepared as is described in chapter 7.1.4.4 "Rezepturbeispiel" on page 205 of the textbook "Kosmetik", W. Umbach (editor), 2nd edition, Thieme Verlag. 1995, wherein, in addition to the ingredients mentioned in said chapter on page 205, the above mentioned microorganism belonging to the group of lactic acid bacteria is added in an amount of 10² to 10¹², preferably 103 to 10⁸ cells per mg of the gel-dentifrice on basis of silicic acid/sodium fluoride.

### Example 24

### Dentifrice composition against tartar

The dentifrice composition against tartar is preferably prepared as is described in chapter 7.1.4.4 "Rezepturbeispiel" on page 206 of the textbook "Kosmetik", W. Umbach (editor), 2nd edition, Thieme Verlag, 1995, wherein, in addition to the ingredients mentioned in said chapter on page 206, the above mentioned microorganism belonging to the group of lactic acid bacteria is added in an amount of 102 to 10¹², preferably 10³ to 10⁸ cells per mg of the dentifrice against tartar.

### Example 25

### Chewing gum composition

The chewing gum composition is preferably prepared as is described in Example 6 on page 9 of DE-C2 36 45 147, wherein, in addition to the ingredients mentioned in said Example 6, the above mentioned microorganism belonging to the group of lactic acid bacteria is added in an amount of 10² to 10¹², preferably 10³ to 10⁸ cells per mg of the chewing gum.

### Example 26

### Concentrated mouthwash composition

The concentrated mouth wash composition is preferably prepared as is described in chapter 7.1.4.4 "Rezepturbeispiel" on page 206 of the textbook "Kosmetik", W. Umbach (editor), 2nd edition, Thieme Verlag, 1995, wherein, in addition to the ingredients mentioned in said chapter on page 206, the above mentioned microorganism belonging to the group of lactic acid bacteria is added in an amount of 10² to 10¹³, cells per ml of the concentrated mouthwash composition.

### Example 27

### Film preparation

### Preparation of films:

1. water phase
   - heat water to 60°C
   - aspartame (sweetener) is added under stirring
   - aspartame is dissolved completely
   - a polymeric water-soluble film former, like, for example, Kollicoat IR (polyethylenglycol on polyvinylalcohol) or PVP (polyvinylpyrrolidon) or natural polymers such as alginates are added under stirring until they are dissolved
   - after 10 min. the rest of the foam is removed
   - the above mentioned microorganism belonging to the group of lactic acid bacteria in an amount of 10² to 10¹², preferably 10³ to 10⁸ cells per final aroma film is added after cooling down of the mixture; alternatively, the mutant or derivative of the above mentioned microorganism belonging to the group of lactic acid bacteria or an analog or fragment of the above mentioned microorganism belonging to the group of lactic acid bacteria can be added
2. oily phase
   - menthol is dissolved in peppermint-oil
   - polysorbat 80 is added to the peppermint-oil - menthol - mix under stirring
   - this mixture is then added to propylene-glykole under stirring
   - optional colorants (such as pigments, lakes) can be added
3.
   - under stirring the oily phase is slowly mixed with the water phase
4.
   - the thin films are mechanically generated using a cutting device

### Sample formulations:

| | **formulation I** | | **formulation II** | |
|---|---|---|---|---|
| | **weight [g]** | **composition in film [%]** | **weight [g]** | **composition in film [%]** |
| **Phase I** | | | | |
| aspartame | 0.7 | 1.4 | 0.7 | 1.8 |
| Kollicoat IR | 35.0 | 68.5 | 25.0 | 65.8 |
| ascorbic acid | - | - | 1.0 | 2.6 |
| cherry flavour | | | 6.0 | 15.8 |
| water demin. | 85.0 | - | 80.0 | |

| **Phase II** | | | | |
|---|---|---|---|---|
| menthol | 1.4 | 2.7 | - | |
| peppermint oil | 5.6 | 11.0 | - | |
| polysorbat 80 | 0.7 | 1.4 | - | |
| propylene glykol | 7.0 | 13.7 | 5.0 | 13.2 |
| green lake | 0.7 | 1.4 | - | |
| azorubin lake | - | - | 0.3 | 0.8 |
| sum | 136.1 | 100.0 | 118.0 | 100.0 |
| solid content | 51.1 | | 38.0 | |

It is intended that the specification and examples be considered exemplary only with the true scope of the invention indicated by the following claims.

## Claims

1. Use of a microorganism belonging to the genus Lactobacillus or a mutant or derivative thereof, **characterized in that** it is capable of specifically binding to a bacterium belonging to the group of mutans Streptococci, wherein the specific binding is
(i) resistant to heat treatment, wherein said heat treatment is carried out at a temperature of more than 95 °C for at least 20 minutes; and
(ii) resistant to protease treatment, wherein said protease treatment is treatment with a protease selected from the group consisting of pronase E, proteinase K, trypsin and chymotrypsin; and
(iii) calcium-dependent; and
(iv) formed within a pH range between 4.5 and 8.5; and
(v) formed in the presence of saliva,
for the preparation of an anticariogenic composition for the treatment or prevention of caries caused by Streptococcus sobrinus by binding to said Streptococcus sobrinus,
wherein said derivative of the microorganism is an inactivated form of said microorganism or a fragment of said microorganism, said microorganism being thermally inactivated or lyophilized, wherein said fragment is a membrane fraction obtained by a membrane preparation and wherein said inactivated form or said fragment retains the capability of specifically binding a bacterium belonging to the group of mutans Streptococci.

2. The use of claim 1, wherein the specific binding can be assayed as follows:
(a) growing said microorganism to stationary phase;
(b) mixing said microorganism with a bacterium belonging to the group of mutans Streptococci which has been grown to stationary phase;
(c) incubating the mixture obtained in step (b) under conditions allowing the formation of aggregates of said microorganism and a bacterium of the group of mutans Streptococci; and
(d) detecting aggregates by the occurrence of a pellet.

3. The use of claim 1 or 2, wherein said microorganism is a Lactobacillus paracasei selected from the group consisting of Lactobacillus paracasei having DSMZ accession number DSM 16667, DSMZ accession number DSM 16668, DSMZ accession number DSM 16669, DSMZ accession number DSM 16670 and DSMZ accession number DSM 16671, or a mutant or derivative thereof, wherein said mutant or derivative retains the capability to bind to a bacterium belonging to the group of mutans Streptococci, or wherein said Lactobacillus is a Lactobacillus rhamnosus selected from the group consisting Lactobacillus rhamnosus having DSMZ accession number DSM 16672 and DSMZ accession number DSM 16673 or a mutant or derivative thereof, wherein said mutant or derivative retains the capability to bind to a bacterium belonging to the group of mutans Streptococci.

4. The use of any one of claims 1 to 3, wherein said microorganism is capable of binding to at least one bacterium selected from the group consisting of Streptococcus mutans, Streptococcus sobrinus, Streptococcus cricatus, Streptococcus ratti, Streptococcus ferus and Streptococcus macacae.

5. The use of claim 4, wherein said microorganism is capable of binding to
(a) Streptococcus mutans serotype c (DSMZ 20523) and/or serotype e (NCTC 10923) and/or serotype f (NCTC 11060), or
(b) Streptococcus sobrinus DSM 20742, or
(c) Streptococcus ratti DSM 20564, or
(d) Streptococcus cricetus DSM 20562, or
(e) Streptococcus ferus DSM 20646, or
(f) Streptococcus macacae DSM 20714.

6. The use of any one of claims 1 to 5, wherein said anticariogenic composition is a cosmetic or pharmaceutical composition, which additionally comprises a cosmetically, pharmaceutically or orally acceptable carrier or excipient.

7. The use of any one of claims 1 to 6, wherein said anticariogenic composition is a dentifrice, chewing gum, lozenge, mouth wash, mouse rinse, dental floss or dental tape, or is an anticariogenic foodstuff or feedstuff, or is an additive for food, feed or drinks.

## Patentansprüche

1. Verwendung eines Mikroorganismus, der zu der Gattung Lactobacillus gehört, oder einer Mutante oder eines Derivats davon, **dadurch gekennzeichnet, dass** er fähig ist, spezifisch an ein Bakterium zu binden, das zu der Gruppe von Mutans-Streptococci gehört, wobei die spezifische Bindung
(i) beständig gegen Wärmebehandlung, wobei die Wärmebehandlung wenigstens 20 Minuten bei einer Temperatur von über 95 °C durchgeführt wird; und
(ii) beständig gegen Protease-Behandlung, wobei die Protease-Behandlung eine Behandlung mit einer Protease, ausgewählt aus der Gruppe bestehend aus Pronase E, Proteinase K, Trypsin und Chymotrypsin ist; und
(iii) Calcium-abhängig; und
(iv) in einem pH-Wert-Bereich zwischen 4,5 und 8,5 gebildet; und
(v) in Gegenwart von Speichel gebildet ist, für die Herstellung einer antikariogenen Zusammensetzung für die Behandlung oder Vorbeugung von Karies, der durch Streptococcus sobrinus verursacht ist, durch Binden an den Streptococcus sobrinus,
wobei das Derivat des Mikroorganismus eine inaktivierte Form des Mikroorganismus oder ein Fragment des Mikroorganismus ist, wobei der Mikroorganismus thermisch inaktiviert oder lyophilisiert ist, wobei das Fragment eine Membranfraktion ist, erhalten durch eine Membran-Aufarbeitung, und wobei die inaktivierte Form oder das Fragment die Fähigkeit zum spezifischen Binden eines Bakteriums, das zu der Gruppe von Mutans-Streptococci gehört, behält.

2. Verwendung gemäß Anspruch 1, wobei das spezifische Binden folgendermaßen geprüft werden kann:
(a) Kultivieren des Mikroorganismus zur stationären Phase;
(b) Mischen des Mikroorganismus mit einem Bakterium, das zu der Gruppe von Mutans-Streptococci gehört, das zur stationären Phase kultiviert worden ist;
(c) Inkubieren des bei Schritt (b) erhaltenen Gemischs unter Bedingungen, die die Bildung von Aggregaten des Mikroorganismus und eines Bakteriums der Gruppe von Mutans-Streptococci erlauben; und
(d) Nachweisen von Aggregaten durch das Auftreten eines Pellets.

3. Verwendung gemäß Anspruch 1 oder 2, wobei der Mikroorganismus ein Lactobacillus paracasei ist, ausgewählt aus der Gruppe bestehend aus Lactobacillus paracasei mit der DSMZ-Hinterlegungsnummer DSM 16667, der DSMZ-Hinterlegungsnummer DSM 16668, der DSMZ-Hinterlegungsnummer DSM 16669, der DSMZ-Hinterlegungsnummer DSM 16670 und der DSMZ-Hinterlegungsnummer DSM 16671, oder eine Mutante oder ein Derivat davon, wobei die Mutante oder das Derivat die Fähigkeit behält, an ein Bakterium zu binden, das zu der Gruppe von Mutans-Streptococci gehört, oder wobei der Lactobacillus ein Lactobacillus rhamnosus ist, ausgewählt aus der Gruppe bestehend aus Lactobacillus rhamnosus mit der DSMZ-Hinterlegungsnummer DSM 16672 und mit der DSMZ-Hinterlegungsnummer DSM 16673 oder eine Mutante oder ein Derivat davon, wobei die Mutante oder das Derivat die Fähigkeit behält, an ein Bakterium zu binden, das zu der Gruppe von Mutans-Streptococci gehört.

4. Verwendung gemäß einem der Ansprüche 1 bis 3, wobei der Mikroorganismus fähig ist, an wenigstens ein Bakterium ausgewählt aus der Gruppe bestehend aus Streptococcus mutans, Streptococcus sobrinus, Streptococcus cricatus, Streptococcus ratti, Streptococcus ferus und Streptococcus macacae zu binden.

5. Verwendung gemäß Anspruch 4, wobei der Mikroorganismus fähig ist, an
(a) Streptococcus mutans Serotyp c (DSMZ 20523) und/oder Serotyp e (NCTC 10923) und/oder Serotyp f (NCTC 11060) oder
(b) Streptococcus sobrinus DSM 20742 oder
(c) Streptococcus ratti DSM 20564 oder
(d) Streptococcus cricetus DSM 20562 oder
(e) Streptococcus ferus DSM 20646 oder
(f) Streptococcus macacae DSM 20714
zu binden.

6. Verwendung gemäß einem der Ansprüche 1 bis 5, wobei die antikariogene Zusammensetzung eine kosmetische oder pharmazeutische Zusammensetzung ist, die zusätzlich einen kosmetisch, pharmazeutisch oder oral verträglichen Träger oder Hilfsstoff umfasst.

7. Verwendung gemäß einem der Ansprüche 1 bis 6, wobei die antikariogene Zusammensetzung ein Zahnputzmittel, ein Kaugummi, eine Lutschtablette, eine Mundwäsche, eine Mundspülung, Zahnseide oder ein Zahnband ist oder ein antikariogenes Lebensmittel oder Futtermittel ist oder ein Zusatzstoff für Lebensmittel, Futtermittel oder Getränke ist.

## Revendications

1. Utilisation d'un micro-organisme appartenant au genre Lactobacillus, ou d'un mutant ou d'un dérivé de ce dernier, **caractérisée en ce qu'**il est capable de se lier spécifiquement à une bactérie appartenant au groupe des Streptococcus mutans, la liaison spécifique
(i) étant résistante à un traitement thermique, ledit traitement thermique étant mis en oeuvre à une température supérieure à 95°C pendant au moins 20 minutes ; et
(ii) étant résistante à un traitement par une protéase, ledit traitement par une protéase étant un traitement par une protéase choisie dans le groupe consistant en la pronase E, la protéinase K, la trypsine et la chymotrypsine ; et
(iii) étant calcium-dépendante ; et
(iv) étant formée sur une gamme de pH comprise entre 4,5 et 8,5 ; et
(v) étant formée en présence de salive,
pour la préparation d'une composition anticariogène pour le traitement ou la prévention de caries provoquées par Streptococcus sobrinus, par liaison audit Streptococcus sobrinus,
ledit dérivé du micro-organisme étant une forme inactivée dudit micro-organisme ou un fragment dudit micro-organisme, ledit micro-organisme étant thermiquement inactivé ou lyophilisé, ledit fragment étant une fraction membranaire obtenue par une préparation membranaire, et ladite forme inactivée dudit fragment conservant la capacité de se lier spécifiquement à une bactérie appartenant au groupe de Streptococcus mutans.

2. Utilisation de la revendication 1, pour laquelle la liaison spécifique peut être analysée comme suit :
(a) culture dudit micro-organisme jusqu'à la phase stationnaire ;
(b) mélange dudit micro-organisme à une bactérie appartenant au groupe de Streptococcus mutans qui a été cultivée jusqu'à la phase stationnaire ;
(c) incubation du mélange obtenu dans l'étape (b) dans des conditions permettant la formation d'agrégats dudit micro-organisme, et d'une bactérie du groupe de Streptococcus mutans ; et
(d) détection des agrégats par l'apparition d'un culot.

3. Utilisation de la revendication 1 ou 2, pour laquelle ledit micro-organisme est un Lactobacillus paracasei choisi dans le groupe consistant en Lactobacillus paracasei ayant le numéro d'accession DSMZ DSM 16 667, le numéro d'accession DSMZ DSM 16 668, le numéro d'accession DSMZ DSM 16 669, le numéro d'accession DSMZ DSM 16 670 et le numéro d'accession DSMZ DSM 16 671, ou un mutant ou un dérivé de ceux-ci, pour laquelle ledit mutant ou dérivé conserve la capacité de se lier à un bactérie appartenant au groupe des Streptococcus mutans, ou pour laquelle ledit Lactobacillus est un Lactobacillus rhamnosus choisi dans le groupe consistant en Lactobacillus rhamnosus ayant le numéro d'accession DSMZ DSM 16 672 et le numéro d'accession DSMZ DSM 16 673 ou un mutant ou un dérivé de ceux-ci, ledit mutant ou dérivé conservant la capacité de se lier à une bactérie appartenant au groupe des Streptococcus mutans.

4. Utilisation de l'une quelconque des revendications 1 à 3, pour laquelle ledit micro-organisme est capable de se lier à au moins une bactérie choisie dans le groupe consistant en Streptococcus mutans, Streptococcus sobrinus, Streptococcus cricatus, Streptococcus ratti, Streptococcus ferus et Streptococcus macacae.

5. Utilisation de la revendication 4, pour laquelle ledit micro-organisme est capable de se lier
(a) à Streptococcus mutans, sérotype c (DSMZ 20 523) et/ou sérotype e (NCTC 10 923) et/ou sérotype f (NCTC 11 060) ou
(b) à Streptococcus sobrinus DSM 20 742, ou
(c) à Streptococcus ratti DSM 20 564, ou
(d) à Streptococcus cricetus DSM 20 560, ou
(e) à Streptococcus ferus DSM 20 646, ou
(f) à Streptococcus macacae DSM 20 714.

6. Utilisation de l'une quelconque des revendications 1 à 5, pour laquelle ladite composition anticariogène est une composition cosmétique ou pharmaceutique, qui en outre comprend un support ou un excipient acceptable d'un point de vue cosmétique, pharmaceutique ou oral.

7. Utilisation de l'une quelconque des revendications 1 à 6, pour laquelle ladite composition anticariogène est un dentifrice, une gomme à mâcher, une tablette, un bain de bouche, un rince-bouche, un fil dentaire ou une bande dentaire à polir, ou un produit anticariogène pour l'alimentation humaine ou animale, ou est un additif pour un aliment pour l'alimentation humaine ou animale, ou pour des boissons.
